# EUROPEAN PATENT SPECIFICATION

(11) **EP 2 601 211 B1**
(45) Date of publication and mention of the grant of the patent: **11.10.2017**
(21) Application number: 11743319.3
(22) Date of filing: 02.08.2011
(51) Int. Cl.: C07K 14/455

(54) **VACCINE**
IMPFSTOFF
VACCIN

(30) Priority: 02.08.2010 GB 201013006
(43) Date of publication of application: 12.06.2013
(73) Proprietor: The Pirbright Institute, Pirbright Woking Surrey GU24 0NF (GB)
(72) Inventor: BLAKE, Damer, Hatfield Hertfordshire AL9 7TA (GB); SMITH, Adrian, Oxford Oxfordshire OX1 3PS (GB); SHIRLEY, Martin, Cambridge Cambridgeshire PE19 5SR (GB)
(74) Representative: D Young & Co LLP
(86) International application number: PCT/GB2011/051456
(87) International publication number: WO 2012/017230

(56) References cited:
- US-A1- 2006 115 498
- DAMER P. BLAKE ET AL: "Genetic Mapping Identifies Novel Highly Protective Antigens for an Apicomplexan Parasite", PLOS PATHOGENS, vol. 7, no. 2, 1 January 2011 (2011-01-01) , page E1001279, XP55009489, ISSN: 1553-7366, DOI: 10.1371/journal.ppat.1001279
- DATABASE EMBL [Online] 14 April 2011 (2011-04-14), "Eimeria maxima mRNA for immune mapped protein 1 (imp-1 gene), strain Weybridge", XP002661344, retrieved from EBI accession no. EMBL:FN813227 Database accession no. FN813227
- BLAKE D P ET AL: "Parasite genetics and the immune host: recombination between antigenic types of Eimeria maxima as an entree to the identification of protective antigens", MOLECULAR AND BIOCHEMICAL PARASITOLOGY, ELSEVIER SCIENCE PUBLISHERS, AMSTERDAM, NL, vol. 138, no. 1, 1 November 2004 (2004-11-01), pages 143-152, XP004611810, ISSN: 0166-6851, DOI: 10.1016/J.MOLBIOPARA.2004.08.006 cited in the application
- DATABASE EMBL [Online] 23 March 2009 (2009-03-23), "04Mar08_P2_RS18_QX_B02 Eimeria maxima merozoite life stage cDNA library Eimeria maxima cDNA similar to Unique, mRNA sequence.", XP002661345, retrieved from EBI accession no. EMBL:GO304933 Database accession no. GO304933
- DATABASE UniProt [Online] 24 March 2009 (2009-03-24), "SubName: Full=Putative uncharacterized protein;", XP002661346, retrieved from EBI accession no. UNIPROT:B9PY37 Database accession no. B9PY37
- SHIRLEY ET AL: "Challenges in the successful control of the avian coccidia", VACCINE, ELSEVIER LTD, GB, vol. 25, no. 30, 10 July 2007 (2007-07-10) , pages 5540-5547, XP022148591, ISSN: 0264-410X, DOI: 10.1016/J.VACCINE.2006.12.030
- DATABASE EMBL [Online] 2 October 2009 (2009-10-02), "Toxoplasma gondii cDNA, clone: XTG07238.2, full cDNA, XTG Sugano cDNA library.", XP002661347, retrieved from EBI accession no. EM_HTG:AK318455 Database accession no. AK318455
- SHIRLEY MARTIN W ET AL: "The biology of avian Eimeria with an emphasis on their control by vaccination", ADVANCES IN PARASITOLOGY, ACADEMIC PRESS, LONDON, GB, vol. 60, 1 January 2005 (2005-01-01), pages 285-330, XP009111817, ISSN: 0065-308X
- DATABASE UniProt [Online] 24 March 2009 (2009-03-24), "SubName: Full=Immune mapped protein 1 {ECO:0000313|EMBL:ESS29244.1}; SubName: Full=Immune mapped protein-1 {ECO:0000313|EMBL:AFB35815.1}; SubName: Full=Uncharacterized protein {ECO:0000313|EMBL:CAJ20428.1};", retrieved from EBI accession no. UNIPROT:B9PY37 Database accession no. B9PY37

## Description

### FIELD OF THE INVENTION

The present invention relates to a polypeptide or nucleic acid useful as a vaccine, or a component of a vaccine, against an apicomplexan parasite.

### BACKGROUND TO THE INVENTION

The protozoan phylum Apicomplexa contains pathogens of medical and veterinary importance including *Toxoplasma gondii* and the *Eimeria, Plasmodium* and *Theileria* species. Despite several decades of effort, vaccines protective against these and other related parasites are scarce. Empiric approaches to identify genuinely immunoprotective antigens as vaccine candidates have achieved mixed results and problems differentiating immunogenicity from real immune protection persist.

There is thus a need to develop improved vaccines against protozoan parasites.

As food security gains in importance, pathogens which impact upon economic poultry production are re-emerging as serious threats to global food supply and human poverty. *Eimeria* species parasites have a globally enzootic distribution and can cause severe enteric disease in all livestock, most notably poultry, where the annual cost is estimated to exceed £2 billion worldwide.

The total cost of coccidial infections in the UK poultry industry (circa 780 million broilers) has been estimated to be at least £42m per annum, of which 74% is due to sub-clinical effects on weight gain and feed conversion and 24% is the cost of prophylaxis and therapy of commercial birds (Williams, 1999; A compartmentalized model for the estimation of the cost of coccidiosis to the world's poultry production industry. International Journal for Parasitology, 1209-1229).

Infections with *Eimeria* spp. in intensively reared poultry have to date been controlled primarily by prophylactic in-feed medication (especially for broiler production). Indeed, more than a third of all chemotherapeutics used in UK animal production are applied to control *Eimeria* (http://www.vmd.gov.uk/), but resistance is rife and alternatives, such as vaccination with attenuated parasites, are limited by the need to include live versions of multiple *Eimeria* spp., production issues and cost.

Shirley et al. 2007 (Vaccine, vol. 25, no. 30, 10 July 2007, pages 5540-5547) provide a review of past and present methods of controlling avian coccidian and describe the difficulty in determining the identity of suitable antigens. Shirley *et al.* describe approaches for identifying immunoprotective antigens as vaccine candidates.

There is thus a need to develop new cost-effective control strategies for *Eimeria* species parasites.

### DESCRIPTION OF THE FIGURES

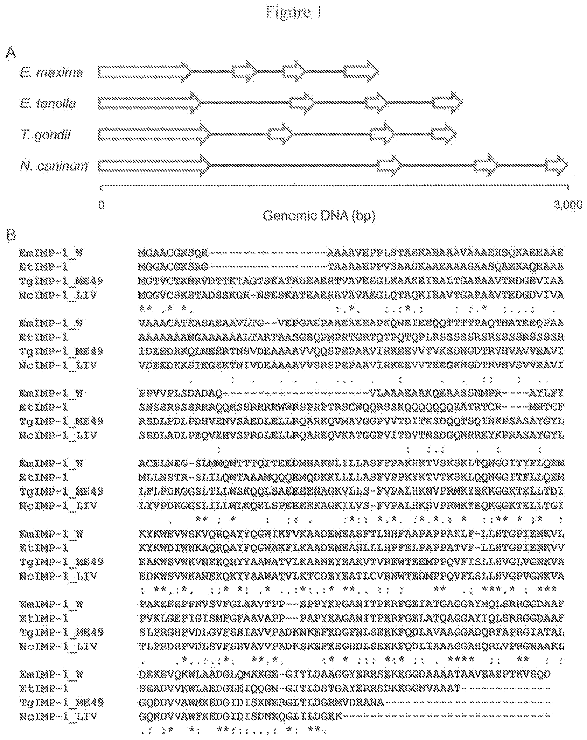

**Figure 1** **-** Putative apicomplexan Immune Mapped Protein-1 (*IMP-1*) homologues. (a) *IMP-1* intron/exon structure from *Eimeria maxima, Eimeria tenella, T. gondii* (XM_002370108) and *Neospora caninum* (NCLIV_000430; GeneDB). (b) Full length IMP-1 alignment using sequenced translated coding sequences.

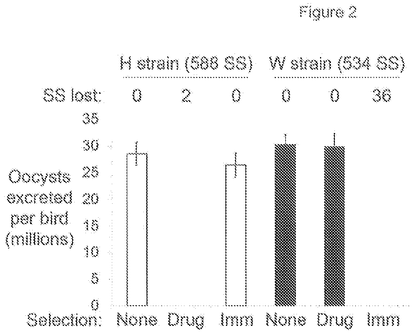

**Figure 2** **-** *Eimeria maxima* strain-specific traits. The influence of drug (robenidine) and W strain-specific immunity on subsequent H and W strain parasite replication and the persistence of strain-specific AFLP markers (SS) in H/W hybrid populations under selection.

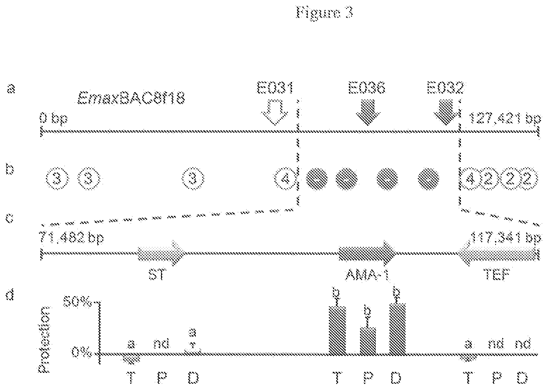

**Figure 3** **-** Fine mapping locus 1 correlated with susceptibility to strain-specific immune killing. **(a)** The distribution of strain-specific AFLP markers lost under immune selection across BAC *Emax*BAC8f18. Solid markers remained absent after six generations of backcrossing under selection. **(b)** Recovery of strain-specific markers by immune selected backcross generations 1-6, the number represents the generation at which the marker first reappeared, - = did not reappear. **(c)** Transcribed sequences identified within locus 1 (ST = putative sulphate transporter, AMA-1 = apical membrane antigen 1 homologue, TEF = putative transcription elongation factor). **(d)** Percentage reduction in oocyst output induced by immunisation with individual genes identified within locus 1 compared to mock immunised controls, candidates presented as an *E. maxima* H vectored transgene compared to no transgene (T), recombinant protein compared to thioredoxin alone (P) or DNA compared to vector only (D) vaccination. nd = not done. Bars marked with different letters were significantly different (p<0.05).

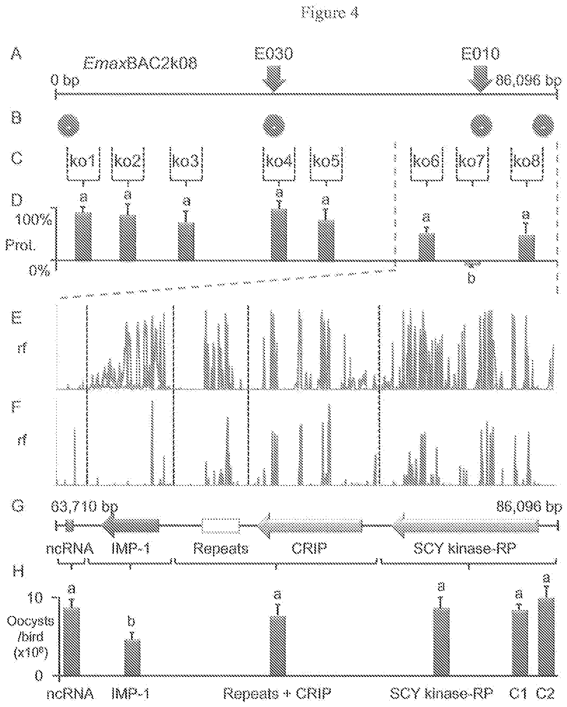

**Figure 4** **-** Fine mapping locus 5 correlated with susceptibility to strain-specific immune killing. **(a)** The distribution of strain-specific AFLP markers lost under immune selection across BAC *Emax*BAC2k08. **(b)** Recovery of strain-specific markers by immune selected backcross generations revealed that the entire BAC encoded-region remained absent in all generations (-). **(c)** Regions 1-8 selected for disruption by BAC recombineering. **(d)** The influence of targeted disruption on the ability of W strain-derived *Emax*BAC2k08 to confer cross-protective immunity when delivered by the heterologous *E. maxima* H strain following transient transfection: the protective capacity retained by each recombineered BAC compared to the unmodified parent BAC. (**e**-**f**) Relative fluorescence (rf) illustrating cDNA hybridisation to a BAC-tiling NimbleGen array covering ∼22.4 Kb of locus 5 corresponding to the smaller *Not* I/*Sfi* I BAC digest product. (e) cDNA derived from sporozoite (blue) and first generation merozoite (red) lifecycle stages. **(f)** Profiles calculated from rf using cDNA derived from uninfected chicken intestinal tissue subtracted from chicken intestinal tissue 6 (blue) and 16 (red) hours post infection. **(g)** Transcribed sequences identified within locus 5 (repeats = a repetitive region, no evidence of transcription by targeted lifecycle stages, CRIP = putative cyclophilin-RNA interacting protein, RP = related protein. **(h)** Average total *E. maxima* W strain oocyst output per bird following challenge of birds immunised with *E. maxima* H vectored W strain transgenes presented as genomic LD-PCR amplicons. C1 = control, no transgene; C2 = control, no immunisation. Bars marked with different letters were significantly different (p<0.05).

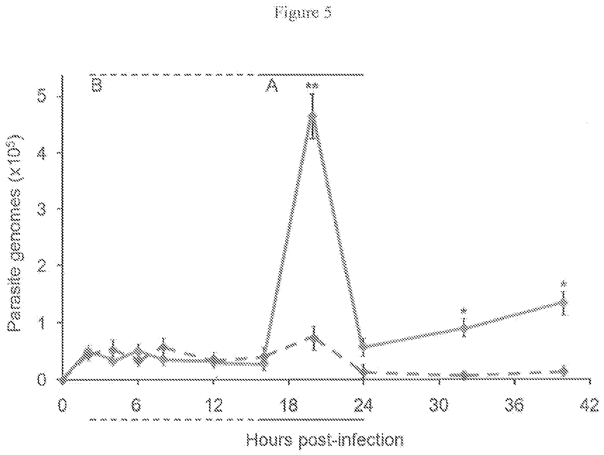

**Figure 5** **-** *In vivo* intracellular *E. maxima* W strain replication in naive (red, solid line) and previously infected (homologous infection; blue, broken line) chickens. Total parasite genome numbers determined for an 8 cm intestinal section covering Meckel's diverticulum. Region A highlights the first significant difference in parasite replication between the immunised and unimmunised host. Importantly, whilst qPCR-detected parasite genome numbers are not equivalent to viable parasites, the residual parasite genomes detected after 20 hpi in the immune host were not seen to replicate and were unlikely to represent live parasites. Thus, regions A and B represent the period when immune killing must have occurred. *p<0.01, **p<0.005, ANOVA + Tukey's post hoc.

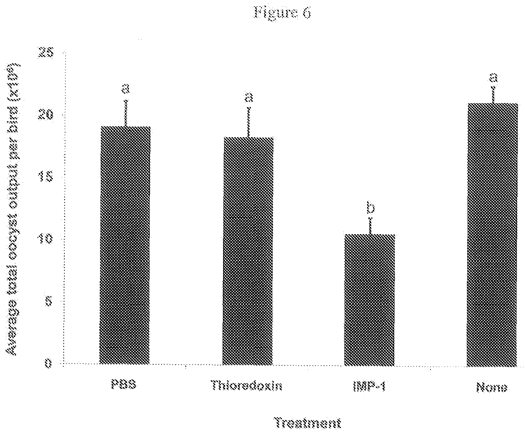

**Figure 6** **-** Anti-coccidial protection induced by vaccination with recombinant EmIMP-1 compared with thioredoxin, PBS and no immunisations (protein, immunisation and environmental controls respectively). Bars marked with different letters were significantly different (p<0.01, ANOVA + Tukey's post hoc).

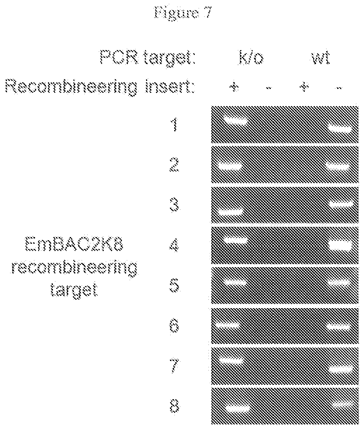

**Figure 7** **-** PCR confirmation of targeted *Emax*BAC2k08 disruption by recombineering for eight selected regions (1-8). PCR assays confirming the targeted insertion of the recombineering cassette (knockout: k/o) and the absence of unmodified BAC copies (wild type: wt). + = recombineered candidate BAC, - = unmodified original BAC.

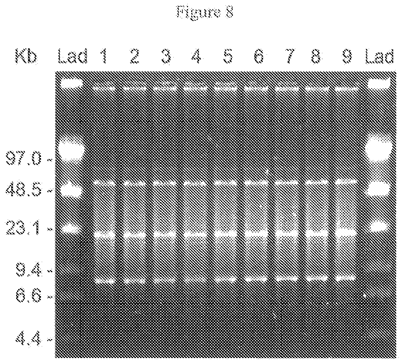

**Figure 8** **-** PFGE resolution of *Not* I/*Sfi* I digested unmodified *Emax*BAC2k08 (lane 1) and *Emax*BAC2k08 recombineered to disrupt regions 1-8 (lanes 2-9).

### SUMMARY OF ASPECTS OF THE INVENTION

The present inventors have identified two genuinely protective anti-coccidial vaccine candidates. The candidates, identified in *Eimeria,* have readily identifiable homologues encoded within the genomes of related apicomplexan parasites.

Thus, in a first aspect, the present invention provides a polypeptide from an *Eimeria* species parasite comprising the amino acid sequence as shown as SEQ ID No. 1 or the *Eimeria tenella* amino acid sequence shown as Et IMP-1 shown in Figure 1 (b) or a variant having at least 80% sequence identity to SEQ ID NO: 1 and being capable of inducing a protective immune response in a subject against subsequent challenge with an apicomplexan parasite.

In a second aspect, the present invention provides a nucleic acid capable of encoding a polypeptide according to the first aspect of the invention. The nucleic acid sequence may comprise the nucleotide sequence shown as SEQ ID No. 2 or 6.

In a third aspect, the present invention provides a vector comprising a nucleic acid according to the second aspect of the invention.

In a fourth aspect, there is provided a polypeptide, nucleic acid or vector according to any of the first three aspects of the invention, for use in the treatment and/or prevention of a disease.

In a fifth aspect, the present invention provides a vaccine for the treatment and/or prevention of a disease associated with an Eimeria species parasite, which comprises:
(i) a polypeptide according to claim 1; and/or
(ii) a nucleic acid sequence capable of encoding such a polypeptide.

Apicomplexan parasites include, but are not limited to, the following group of species: *Eimeria, Neospora, Plasmodium, Theileria* and *Toxoplasma.*

The disease may be selected from, but is not limited to, the following: babesiosis, coccidiosis cryptosporidiosis, cyclosporiasis, isosporiasis, malaria and toxoplasmosis.

The vaccine may comprise a plurality of amino acid/nucleic acid sequences corresponding to SEQ ID No. 1 homologues from different species of apicomplexan parasite.

For example, a vaccine against *Eimeria* infection in chickens may comprise a plurality of amino acid/nucleic acid sequences corresponding to SEQ ID No. 1 homologues from two or more of the following species: *Eimeria acervulina; Eimeria brunetti; Eimeria maxima; Eimeria mitis; Eimeria necatrix; Eimeria praecox and Eimeria tenella.*

The vaccine may also comprise:
(i) an amino acid sequence as shown as SEQ ID No. 3 or a homologue or fragment thereof; and/or
(ii) a nucleic acid sequence capable of encoding an amino acid sequence as shown as SEQ ID No. 3 or a homologue thereof.

The disease may be coccidiosis.

The vaccine may comprise a plurality of amino acid/nucleic acid sequences corresponding to SEQ ID No. 3 homologues from different *Eimeria* species and strains.

Disclosed herein is a method for treating and/or preventing a disease in a subject by administration of an effective amount of a polypeptide according to the first aspect of the invention, nucleic acid according to the second aspect of the invention, any vector according to the third aspect of the invention or a vaccine according to the fifth aspect of the invention, to the subject.

The subject may be a vertebrate subject. The subject may, for example, be a mammalian or avian subject. The subject may be a poultry subject, such as a bantam, chicken, duck, goose, guinea fowl, partridge, pheasant or turkey.

### DETAILED DESCRIPTION

### POLYPEPTIDE

The term "polypeptide" is used in the normal sense to mean a series of residues, typically L-amino acids, connected one to the other typically by peptide bonds between the α-amino and carboxyl groups of adjacent amino acids. The term is synonymous with "protein". The polypeptide may be isolated from its natural environment, for example, extracted from or produced outside of an apicomplexan parasite.

The first aspect of the invention relates to a polypeptide from an *Eimeria* species parasite comprising the amino acid sequence as shown as SEQ ID No. 1 or the *Eimeria tenella* amino acid sequence shown as Et IMP-1 shown in Figure 1 (b) or a variant having at least 80% sequence identity to SEQ ID NO: 1 and being capable of inducing a protective immune response in a subject against subsequent challenge with an apicomplexan parasite.
SEQ ID No.1: IMP-1 *E. maxima* Weybridge strain translated cDNA

MGAACGKSQRAAAAVEPPLSTAEKAEAAAVAAAEHSQKAEEAAEVAAACAT
KASAEAAVLTGVEPGAEPAAEAEEAPKQNEIEEQQTTTTPAQTHATEEQPAAP
PVVPLSDADAQVLAAAEAAKQEAASSNMPRAYLFYACELNEGSLMMQWTTT
QITEEDMHAKNLILLASFFPAKHKTVSKSKLTQNGGITYFLQEMKYKWEVWSK
VQRQAYYQGWIKFVKAADEMEASFTLHHFAAPAPPAKLFLLHTGPIENKVLPA
KEEEPFNVSVFGLAAVTPPSPPYKPGANITPKRFGEIATGAGGAYMQLSRRGGD
AAFDEKEVQKWLAADGLQMKKGEGITLDAAGGYERRSEKKGGDAAAATAA
VEAEPTKVSQD

The polypeptide shown as SEQ ID No. 1 is derivable from the *Eimenia maxima* Weybridge (W) strain. Immunisation of chickens with this protein has been shown to induce protection against challenge by the W strain (see Examples).

The corresponding sequence from the *Eimeria maxima* Houghton (H) strain is shown as SEQ ID No. 5. Comparison between the H and W coding sequences uncovered five nucleotide polymorphisms, two of which yield non-synonymous changes.
SEQ ID No. 5: IMP-1 *E. maxima* Houghton strain translated cDNA

MGAACGKSQRAAAAVEPPLSTAEKAEAAAVAAAEHSQKAEEAAEVAAAC
ATKASAEAAVLTGVEPGAEPAAEAEEAPKQNEIEEQQTTTSPAQTHATEEQ
PAAPPVVPLSDADAQVLAAAEAAKQEAASSNMPRAYLFYACELNEGSLM
MQWTTTQITEEDMHAKNLILLASFVPAKHKTVSKSKLTQNGGITYFLQEM
KYKWEVWSKVQRQAYYQGWIKFVKAADEMEASFTLHHFAAPAPPAKLFL
LHTGPIENKVLPAKEEEPFNVSVFGLAAVTPPSPPYKPGANITPKRFGEIATG
AGGAYMQLSRRGGDAAFDEKEVQKWLAADGLQMKKGEGITLDAAGGYE
RRSEKKGGDAAAATAAVEAEPTKVSQD

It is readily possible to identify homologues of this protein within the genome of other apicomplexan parasites. The amino acid sequences for homologues from *E. tenella, T. gondii* and *N. caninum* are shown in Figure 1. As other genomic and/or transcriptomic resources become available it will be possible to identify strain and species specific homologous sequences from other *Eimeria* spp. and from other apicomplexa.

Homologues may, for example, be identified by tBLASTx sequence analysis using the genomic or cDNA sequences, followed by RT-PCR, cloning and sequencing. Other methods include searching based upon structural features of all or part of the molecule.

The vaccine of the present invention may also comprise a polypeptide having the amino acid sequence shown as SEQ ID No. 3, or a homologue thereof.

The polypeptide shown as SEQ ID No. 3, is the *Eimeria maxima* W strain apical membrane antigen-1 (AMA-1). AMA-1 homologues from other apicomplexan parasites have also been identified elsewhere (i.e. Richie and Saul, 2002, Progress and challenges for malaria vaccines, Nature 415: 694-701; Zhang et al., 2010, Neospora caninum: Application of apical membrane antigen 1 encapsulated in the oligomannose-coated liposomes for reduction of offspring mortality from infection in BALB/c mice, Exp Parasitol 125: 130-136).
SEQ ID No.3: AMA-1 *E. maxima* Weybridge strain translated cDNA

MCGLRAAFTEAVCLGLLSLGSTVVQGIKDKVHQGHTEAAATAAAGNLSAE
LHAALHQPNDNPFLVPPLSDFMDRFNIPKVHGSGIYVDLGGDKEVDGRTYR
EPSGLCPVFGKTIVLYQPQNNPNYKNDFLDDIPTKQQSDAVGHPLPGGFNN
SFKMPDKSPYSPMSAQKLNSYKQLKANTPMGKCAEMSFMTTAGKNSSYR
YPWVYDTKRDLCYFLYLPVQRLMGERYCSVDGKPDGMTWYCFEPHKAL
DSRPELVYGSAYVGRDPDYWETHCPNKAVKDAVFGVWVSGRCTEHKHLD
GAKKEKVNSKAECWSLAFENPEVASDHPVTEDENFGTYGYFFPSTEPNQPK
SGGEGVNFASFYPGSMECWLSGEIPTCLVPLEGAAAFTALGSLEEETAPCTD
SFPQTKTPCDRNTCTQIVATCVSGTLVSEEVPCSPEDGTRCEGGFPKGVMIG
LAAAGGILLLLLGGGGFLLYRSRMRPAAKGDEATRSDYVQEEAAANRRKQ
RQSDLVQQAEPSFWEEAEADEAETGESTHVLVDQDY

The polypeptide is or comprises at least part of the wild-type protein (e.g. the protein having the sequence shown as SEQ ID No. 5) or a mutant, variant, homologue or fragment thereof.

The term "wild type" is used to mean a polypeptide having a primary amino acid sequence which is homologous with the native protein (i.e., the protein from the apicomplexan parasite).

The term "mutant" is used to mean a polypeptide having a primary amino acid sequence which differs from the wild type sequence by one or more amino acid additions, substitutions or deletions. A mutant may arise naturally, or may be created artificially (for example by site-directed mutagenesis). The mutant may have at least 80% sequence identity with the wild type sequence and/or up to and including 20 amino acid substitutions, deletions or insertions.

The term "variant" is used to mean a naturally occurring polypeptide which differs from a wild-type sequence. A variant may be found within the same parasitic strain (i.e. if there is more than one isoform of the protein) or may be found within different strains. The variant may have at least 80% sequence identity with the wild type sequence and/or up to and including 20 amino acid substitutions, deletions or insertions.

Here, the term "homologue" means a polypeptide derivable from another strain or species, which performs the same function as the original protein, and/or can be predicted to share a common evolutionary history in terms of their sequence.

A homologous protein (or fragment thereof) may be identified by sequence homology (including the location of critical residues), structural, biophysical biochemical or functional homology.

A homologous sequence may be identified by a conserved putative structure or similarity between amino acid sequences such that alignment using BLASTp comparison yields an E value of 1e-20 or less across a significant portion of the full sequence.

A homologous sequence may be identified by similarity between amino acid sequences or a conserved putative structure. For example, functionally validated homologues to SEQ ID No. 3 specific to *Plasmodium falciparum* and *T. gondii* share only 29% amino acid homology, but retain the same number of cysteine residues in a similar sequence position and consequentially can be predicted to have a conserved protein structure. Typically, the homologues will include some critical sites (e.g. active sites) or structural conservation

Identity or similarity comparisons can be conducted by eye, or more usually, with the aid of readily available sequence comparison programs. These commercially available computer programs, such as: the GCG Wisconsin Bestfit package; the BLAST and FASTA packages; and the GENEWORKS suite of comparison tools, can calculate % identity between two or more sequences.

The term "fragment" indicates that the polypeptide comprises a fraction of the wild-type amino acid sequence. It may comprise one or more large contiguous sections of sequence or a plurality of small sections. The polypeptide may also comprise other elements of sequence, for example, it may be a fusion protein with another protein. The fragment may comprise at least 50%, more preferably at least 65%, most preferably at least 80% of the wild-type sequence. The fragment may be or comprise a sub-unit of the subject sequence.

With respect to function, the mutant, variant, homologue or fragment should be capable of inducing a protective immune response in a subject, against subsequent challenge with the relevant apicomplexan parasite.

### NUCLEIC ACID

In a second aspect, the present invention provides a nucleic acid capable of encoding a polypeptide according to the first aspect of the invention.

The nucleic acid may be any suitable type of nucleotide sequence, such as a synthetic RNA/DNA sequence, a recombinant RNA/DNA sequence (i.e. prepared by use of recombinant DNA techniques), a cDNA sequence or a partial genomic DNA sequence. The nucleic acid may be isolated from its natural environment, for example, extracted from, or produced outside of, an apicomplexan parasite.

The nucleic acid may comprise the coding part of nucleotide sequence shown as SEQ ID No. 2.
SEQ ID No. 2: IMP-1 *E*. *maxima* Weybridge strain cDNA

gcacttttcgtgcattaaatcgctacaattaatttggtagcccgtttttcttctttttcctcttctttggctgtactgtgagggtgg
gttctcccgcgtaggctcatgcagctgttcatactggtctaggcagtgtgcgcctgtgcacgttttgcaagaggatgcatg
cgtttgcgtttcttctttttctgacttttaactctttattacgcgaaacccaaaccacctttactgtctcatccccccttgctgcct
gaggATGGGGCCGCTTGCGGAAATCGCAGCGCCGCCGCTGTTG
AACCCCCCCTTTCTACCGCGGAGAAGGCAGAAGCAGCAGTAGCAG
CAGCAGAACATAGCCAGAAAGCAGAaGAAGCAGCAGAAGTTGCTGCTG
CTTGTGCGACGAAAGCTTCCGCAGAGGCTGCTGTCCTTACAGGGGTaGA
GCCAGGGGCAGAGCCTGCTGCTGAGGCAGAAGAGGCCCCAAAACAAAA
TGAAATAGAAGAGCAGCAAACAACAACAACACCCGCACAAACGCATGC
AACAGAAGAGCAGCCAGCAGCTCCTCCTGTTGTTCCCTTGAGCGATGCA
GATGCACAAGTTCTTGCTGCAGCAGAAGCAGCAAAGCAGGAAGCAGCT
AGtAGCAATATGCCTAGGGCGTATTTGTTTTATGCATGCGAACTCAATGA
AGGATCCTTAATGATGCAATGGACTACTACACAAATAACGGAAGAAGA
TATGCATGCAAAGAATTTAATTCTTCTTGCCTCCTTTTTTCCTGCTAAAC
ATAAAACTGTTTCTAAGAGCAAACTTACACAAAATGGAGGTATTACCTA
CTTCCTGCAGGAGATGAAGTACAAGTGGGAGGTTTGGAGCAAAGTGCA
GAGACAGGCATATTATCAGGGTTGGATAAAATTCGTCAAAGCAGCAGA
TGAGATGGAGGCGTCATTCACCTTACACCACTTTGCTGCTCCTGCGCCA
CCCGCCAAACTTTTCTTACTGCATACAGGACCTATTGAGAATAAGGTAC
TGCCTGCTAAGGAAGAAGAACCATTCAATGTCTCCGTCTTCGGTCTTGC
TGCGGTGACGCCGCCGTCGCCTCCTTACAAGCCAGGAGCGAACATTACA
CCAAAGAGATTCGGCGAAATCGCCACCGGAGCAGGTGGGGCTTACATG
CAGCTTTCCCGCAGAGGCGGTGATGCAGCATTTGATGAGAAAGAAGTTC
AGAAGTGGCTGGCGGCTGACGGTCTTCAAATGAAAAAGGAGAAGGAA
TTACATTGGATGCAGCAGGCGGTTATGAACGAAGATCTGAGAAAAAGG
GGGGCGATGCTGCAGCTGCAACTGCAGCCGTAGAAGCAGAGCCCACTA
AAGTGTCGCAAGATTGA

The start codon is underlined, and the stop codon is shown in bold.

The nucleic acid shown as SEQ ID No. 2 codes for IMP-1 and is derivable from the *Eimeria maxima* Weybridge (W) strain.

The corresponding sequence from *Eimeria* Houghton (H) strain is shown as SEQ ID No. 6. Comparison between the H and W coding sequences uncovered five nucleotide polymorphisms, two of which yield non-synonymous changes.
SEQ ID No. 6: IMP-1 *E. maxima* Houghton strain cDNA

ATGGGGGCCGCTTGCGGGAAATCGCAGCGCGCCGCCGCTGTTGAA
CCCCCCCTTTCTACCGCGGAGAAGGCAGAAGCAGCAGCAGTAGCAGCA
GCAGAACATAGCCAGAAAGCAGAGGAAGCAGCAGAAGTTGCTGCTGCT
TGTGCGACGAAAGCTTCCGCAGAGGCTGCTGTCCTTACAGGGGTCGAGC
CAGGGGCAGAGCCTGCTGCTGAGGCAGAAGAGGCCCAAAACAAAATG
AAATAGAAGAGCAGCAAACAACAACATCACCCGCACAAACGCATGCAA
CAGAAGAGCAGCCAGCAGCTCCTCCTGTTGTTCCCTTGAGCGATGCAGA
TGCACAAGTTCTTGCTGCAGCAGAAGCAGCAAAGCAGGAAGCAGCTAG
CAGCAATATGCCTAGGGCGTATTTGTTTTATGCATGCGAACTCAATGAA
GGATCCTTAATGATGCAATGGACTACTACACAAATAACGGAAGAAGAT
ATGCATGCAAAGAATTTAATTCTTCTTGCCTCCTTTGTTCCTGCTAAACA
TAAAACTGTTTCTAAGAGCAAACTTACACAAAATGGAGGTATTACCTAC
TTCCTGCAGGAGATGAAGTACAAGTGGGAGGTTTGGAGCAAAGTGCAG
AGACAGGCATATTATCAGGGTTGGATAAAATTCGTCAAAGCAGCAGAT
GAGATGGAGGCGTCATTCACCTTACACCACTTTGCTGCTCCTGCGCCAC
CCGCCAAACTTTTCTTACTGCATACAGGACCTATTGAGAATAAGGTACT
GCCTGCTAAGGAAGAAGAACCATTCAATGTCTCCGTCTTCGGTCTTGCT
GCGGTGACGCCGCCGTCGCCTCCTTACAAGCCAGGAGCGAACATTACAC
CAAAGAGATTCGGCGAAATCGCCACCGGAGCAGGTGGGGCTTACATGC
AGCTTTCCCGCAGAGGCGGTGATGCAGCATTTGATGAGAAAGAAGTTC
AGAAGTGGCTGGCGGCTGACGGTCTTCAAATGAAAAAGGGAGAAGGAA
TTACATTGGATGCAGCAGGCGGTTATGAACGAAGATCTGAGAAAAAGG
GGGGCGATGCTGCAGCTGCAACTGCAGCCGTAGAAGCAGAGCCCACTA
AAGTGTCGCAAGATTGA

The corresponding sequence from *Toxoplasma gondii* is shown as SEQ ID No. 7.
SEQ ID No. 7: IMP-1 *T. gondii* cDNA

ATGGGAACCGTTTGCACGAAGAACAGAGTGGACACAACCAAGACAGCT
GGCACCTCGAAAGCAACAGCTGACGAGGCTGAGCGAACAGTCGCTGTT
GAGGAGGGTCTTAAAGCAGCCAAGGAAATTGAGGCTTTGACAGGTGCT
CCCGCTGCGGTTACCAGGGACGGAGAAGTTATCGCAGCGATCGATGAA
GAAGATCGGAAGCAACTGAATGAAGAACGAACGAACAGCGTCGATGA
AGCAGCTGCTGCTGTGGTCCAGCAGTCTCCGGAACCGGCTGCGGTAATT
CGCAAGGAAGAAGTCGTTACAGTAAAGAGCGATAATGGAGACACTCGT
GTCCATGTAGCTGTTGTGGAAGCGGTTATCAGAAGTGATCTGCCGGATC
TTCCTGATCATGTCGAGAATGTTTCGGCTGAGGATCTGGAACTGCTTCG
TCAGGCTCGCAAGCAGGTGATGGCGGTAGGCGGGCCTGTTGTTACGGA
CATCACGAAGTCGGATCAGCAGACCTCCCAAATCAACAAACCAAGTGC
GTCGGCTTACGGGTACCTTCTGTTTTTGCCTGACAAGGGCGGAAGTCTG
ACCCTTCTTTGGTCGAAGCAACAGCTCTCTGCAGAGGAGGAGGAGAAC
GCTGGCAAAGTCCTCCTGTCTTTTGTTCCTGCTCTCCATAAAAACGTCCC
TCGGATGAAATATGAAAAGAAGGGTGGAAAACGGAGCTGCTGACCGA
CATCGAAGCGAAGTGGAGCGTGTGGAAGGTGAATGAGAAGCAGAGGTA
CTACGCGGCATGGGCTACTGTTCTGAAGGCAGCTAACGAATACGAGGC
GAAAGTGACTGTCAGGGAGTGGACTGAAGAAATGCCTCCACAGGTGTT
TATTTCTCTTCTTCATGTTGGACTGGTTGGCAACAAGGTGGCGTCGCTCC
CGCGGGGCCATCCAGTGGATCTTGGCGTTTTCTCGCACATCGCCGTTGTT
CCTGCAGACAAAAACAAAGAGTTCAAAGACGGTTTCAATCTTTCAGAG
AAGAAGTTCCAAGACTTAGCAGTGGCAGCAGGCGGCGCTGATCAGCGA
TTTGCTCCCAGAGGAATCGCCACGGCGTTGGGTCAGGATGATGTGGTAG
CTTGGATGAAAGAAGACGGTATTGATATTAGTAAGAACGAAAGGGGAT
TAACCCTTGATGGCCGAATGGTGGACAGAGCAAATGCGTAG

The corresponding sequence from *Neospora canium* is shown as SEQ ID No. 8.
SEQ ID No. 8: IMP-1 *N. caninum* cDNA

ATGGGAGGCGTTTGCTCTAAAAGCACAGCGGATTCATCCAAGGGTCGTA
ACAGTGAGTCGAAAGCAACCGAGGCTGAGCGGCGGTTGCAGTGGCGG
AGGGTCTTCAAACCGCGCAGAAAATTGAGGCTGTGACAGGCGCTCCCG
CCGCTGTTACCGAGGACGGAGACGTTATCGTGGCCGTAGACGAAGAAG
ATAAGAAATCGATAAAGGGCGAAAAAACGAACATTGTTGACGAAGCAG
CAGCTGCTGTGGTCTCACGTTCTCCCGAACCGGCTGCGGTGATTCGCAA
AGAAGAGGTCGTCACAGAAGAAGGCAAAAATGGAGACACCCGAGTCC
ACGTATCTGTTGTGGAAGCAGTGATCAGTAGCGATCTGGCTGATCTTCC
TGAACAAGTCGAGCATGTTTCGCCCAGGGATTTGGAACTGCTTCGGCAG
GCTCGCGAGCAGGTGAAGGCGACTGGGGGACCCGTTATTACGGATGTC
ACAAACTCAGATGGGCAAAACCGCCGAGAGTACAAACCGAGGGCTTCG
GCTTACGGGTACCTTCTGTACGTGCCTGACAAGGGCGGAAGCCTGATCC
TCCTTTGGTTGAAGCAAGAGCTTTCTCCTGAGGAGGAGGAGAAAGCTGG

CAAAATCCTTGTGTCTTTCGTCCCTGCTCTCCATAAAAGCGTCCCCCGGA
TGAAGTACGAACAGAAGGGTGGAAGACGGAGCTTCTGACCGGCATTG
AAGACAAATGGAGCGTGTGGAAGGCGAATGAGAAACAAAAGTATTACG
CCGCGTGGGCTACGGTTTTAAAGACGTGCGATGAATACGAGGCGACTTT
GTGTGTCCGGAACTGGACAGAAGACATGCCTCCGCAGGTTTTTCTTTCT
CTTCTCCACGTTGGGCCGGTTGGAAACAAGGTGGCAACCCTTCCACGGG
ACCGTCCCGTGGACCTCAGCGTTTTCTCGCACGTCGCCGTTGTTCCGGCA
GATAAAAGCAAAGAGTTCAAAGAGGGCCACGACCTGTCAGAGAAAAA
GTTCCAAGATTTAATAGCGGCAGCAGGAGGCGCCCATCAGCGACTTGTT
CCCAGAGGAAACGCAGCGAAGTTGGGTCAGAATGATGTTGTAGCTTGG
TTCAAAGAAGATGGAATTGATATTAGTGACAACAAGCAAGGACTCATC
CTCGACGGCAAGAAATAG

The vaccine of the present invention may also comprise a nucleic acid sequence capable of encoding a polypeptide having the amino acid sequence shown as SEQ ID No. 3, or a homologue thereof.

The polypeptide shown as SEQ ID No. 3, is the *Eimeria maxima* W strain apical membrane antigen-1 (AMA-1).

The nucleic acid may comprise the coding part of the nucleotide sequence given as SEQ ID No. 4.
SEQ ID No. 4: AMA-1 *E. maxima* Weybridge strain cDNA

CGCGTTGTTTCCCTCTGCTTTTCATTTGAAGTGCTTTCTGCGAAGGG
GATTTAATTCATAACTCTTTCGACAACTCGCAAGTCCCTTTTTGCTTCT
TCTTCAGGAGTCTGGAAACCTGTGCGTCGAGGCACAGCCGGAACACGT
GCAAGGGAGAACGAATCAACAACACCTCGTTTACGTTGCTCTCTCCGTT
GGTTGAGTGGGGCTACTTAGTCTCTGCTTGTTGCTTATTCGCCATTAGTT
CGGACATATTCAGGCTTTTTCACGCCTCCTTGTACCAGCAGGCAGGTGG
AGGCCAGCTGTCTCTGGCCTCCTTGCTTCTTGCATCGGCTTTTGCCTTTA
AGGCATCCCTTGATCTCTTTGAATCTCGTCATCACGGTGTTTTCCTTTAT
TTCATCTTTATTTGTTTTCAAGATGTGTGGATTGCGCGCTGCGTTTACGG
AGGCGGTTTGCTTGGGCCTGCTTTCGTTAGGCTCCACTGTAGTGCAGGG
AATCAAAGATAAAGTTCACCAGGGTCACACAGAAGCAGCAGCAACAGC

AGCAGCAGGAAATCTGTCGGCAGAGCTCCATGCAGCGCTTCACCAGCC
CAACGATAACCCCTTTCTAGTGCCTCCTCTCAGCGATTTCATGGATCGAT
TTAACATCCCGAAGGTCCACGGTAGCGGTATTTACGTAGATCTAGGAGG
CGACAAGGAAGTGGACGGGCGGACATACAGGGAACCGAGCGGCCTCTG
CCCTGTCTTTGGCAAGACGATTGTGTTGTACCAGCCGCAAAACAACCCT
AATTATAAGAATGACTTTCTTGACGACATACCCACCAAACAGCAGTCCG
ATGCCGTAGGACATCCTCTTCCTGGCGGGTTCAATAACAGCTTCAAAAT
GCCAGACAAAAGCCCTTACTCTCCGATGAGTGCACAGAAGTTAAACAG
CTATAAACAGCTGAAGGCGAATACACCGATGGGAAGTGTGCAGAGAT
GAGTTTTATGACGACTGCTGGAAAGAATTCTTCGTATCGGTACCCTTGG
GTCTACGATACGAAACGAGACTTGTGCTATTTCCTCTATCTTCCCGTGCA
ACGGCTCATGGGGGAGCGCTACTGCTCCGTAGATGGCAAACCCGATGG
GATGACGTGGTACTGTTTCGAGCCCCACAAAGCGCTAGATAGCCGACCA
GAACTCGTTTATGGGTCGGCTTACGTAGGAAGGACCCAGACTATTGGG
AAACACACTGCCCCAACAAGGCCGTGAAGGACGCTGTCTTCGGCGTAT
GGGTTTCGGGCAGATGCACAGAGCACAAACACCTCGACGGAGCGAAGA
AGGAAAAAGTGAATAGCAAAGCCGAGTGCTGGTCGCTGGCATTTGAAA
ACCCTGAAGTGGCTAGCGACCATCCTGTAACTGAAGACGAAAACTTCG
GAACATATGGATATTTTTCCCCAGTACCGAGCCCAATCAACCCAAGTC
GGGCGGCGAGGGTGTGAATTTTGCGAGCTTTTATCCGGGTTCAATGGAG
TGCTGGTTATCCGGCGAAATTCCAACTTGTTTGGTGCCTCTTGAGGGGG
CGGCTGCATTCACTGCTCTGGGTTCATTAGAGGAAGAGACGGCACCCTG
TACAGACAGCTTCCCGCAAACGAAGACCCCTTGCGATCGAAACACCTGC
ACACAGATCGTCGCTACATGCGTCTCAGGAACTCTCGTGTCAGAAGAAG
TACCCTGCTCACCAGAGGACGGCACCCGGTGTGAAGGCGGCTTTCCCAA
GGGGGTCATGATAGGTTTGGCAGCAGCGGAGGCATTTTGTTACTGCTG
TTGGGTGGAGGGGGCTTCTTGCTCTATCGTAGCAGAATGAGGCCTGCAG
CGAAGGGAGACGAAGCAACTCGTTCAGATTATGTGCAGGAGGAGGCGG
CAGCCAACAGACGCAAGCAAAGACAGTCGGATTTAGTGCAGCAGGCAG
AGCCTTCCTTCTGGAGGAGGCAGAGGCTGATGAAGCAGAAACGGGGG
AGAGCACCCACGTGTTAGTTGACCAAGATTACTAGTAATAGAGAGGAG
AGAAAAAAAAAAAAAAAAAAAAAAAAAAAA

The start codon is underlined, and the stop codon is shown in bold.

The nucleic acid may comprise the coding sequence part of the sequences shown as SEQ ID No. 4, or a mutant, variant, homologue or fragment thereof.

The term "mutant" is used to mean a nucleotide sequence which differs from the subject sequence by one or more base additions, substitutions or deletions. A mutant may arise naturally, or may be created artificially (for example by site-directed mutagenesis). The mutant may have at least 80% sequence identity with the subject sequence or up to and including 20 mutations. The mutation(s) may be "in frame" so there is not a frameshift over a significant proportion of the encoded sequence.

The term "variant" is used to mean a naturally occurring nucleic acid sequence which differs from a subject sequence. A variant may be found within the same parasitic strain (e.g. a splice variant) or may be found within different strains. The variant may have at least 80% sequence identity with the subject sequence or up to and including 20 mutations.

Here, the term "homologue" means a nucleic acid derivable from another genus, strain or species, which encodes a protein that performs a similar function as the subject protein, and/or can be predicted to share a common evolutionary history in terms of its sequence.

In the present context, a homologous sequence is taken to include a nucleotide sequence which may be identified by direct comparison, similarity between amino acid sequences when translated or a conserved putative protein structure. For example, functionally validated homologues to SEQ ID No. 3 specific to *Plasmodium falciparum* and *T. gondii* share only 29% amino acid homology, but retain the same number of cysteine residues in a similar sequence position and consequentially can be predicted to have a conserved protein structure. Typically, the homologues will include some critical sites (e.g. active sites) or structural conservation as the subject amino acid sequence.

### VECTOR

The present invention also provides a vector comprising a nucleic acid of the present invention.

As used herein, a "vector" may be any agent capable of delivering or maintaining nucleic acid in a host cell, and includes viral, bacterial and eukaryotic vectors, plasmids, naked nucleic acids, nucleic acids complexed with polypeptide or other molecules and nucleic acids immobilised onto solid phase particles.

Nucleic acids in accordance with the present invention can be delivered by viral or non-viral techniques.

Non-infectious delivery systems include but are not limited to DNA transfection methods. Here, transfection includes a process using a non-infectious vector to deliver nucleic acid of the invention to a target cell.

Typical transfection methods include electroporation, nucleic acid biolistics, lipid-mediated transfection, compacted nucleic acid-mediated transfection, liposomes, immunoliposomes, lipofectin, cationic agent-mediated, cationic facial amphiphiles (CFAs), multivalent cations such as spermine, cationic lipids or polylysine, 1, 2,-bis (oleoyloxy)-3-(trimethylammonio) propane (DOTAP)-cholesterol complexes and combinations thereof.

Non-viral delivery systems may also include, but are not limited to, bacterial delivery systems. Bacteria have previously been used as anticancer agents and as delivery agents for anticancer drugs.

Cell adhesion molecules are a large group of molecules involved in a variety of cell-to-cell and cell-to-extra-cellular matrix (ECM) interactions and are exploited by a number of pathogenic micro-organisms as receptors for cell entry. These molecules may be used for the targeting and uptake of both gene and drug delivery systems.

A gene gun delivery system may also be used for the delivery of DNA.

Viral delivery systems include but are not limited to adenovirus vectors, adeno-associated viral (AAV) vectors, herpes viral vectors, retroviral vectors, lentiviral vectors or baculoviral vectors, venezuelan equine encephalitis virus (VEE), poxviruses such as: canarypox virus (Taylor et al 1995 Vaccine 13:539-549), entomopox virus (Li Y et al 1998 XIIth International Poxvirus Symposium p144. Abstract), penguine pox (Standard et al. J Gen Virol. 1998 79:1637-46) alphavirus, and alphavirus based DNA vectors.

Other examples of vectors include *ex vivo* delivery systems, which include but are not limited to DNA transfection methods such as electroporation, DNA biolistics, lipid-mediated transfection and compacted DNA-mediated transfection.

The vector may be a plasmid DNA vector. As used herein, "plasmid" refers to discrete elements that are used to introduce heterologous DNA into cells for either expression or replication thereof.

### APICOMPLEXAN PARASITE

The fourth aspect of the invention relates to a polypeptide, nucleic acid or vector according to any of the first three aspects of the invention, for use in the treatment and/or prevention of a disease associated with an Eimeria species parasite.

The Apicomplexa are a phylum of diverse obligate intracellular parasites including *Plasmodium spp.,* the cause of malaria; as well as the *Cryptosporidium spp., Eimeria spp., Theileria spp.* and *Toxoplasma gondii,* parasites of considerable medical, zoonotic and/or agricultural importance.

The Apicomplexa are protozoa with a structure called the apical complex involved in penetrating a host's cell and most possess an organelle called the apicoplast.

Most members have a complex life-cycle, involving both asexual and sexual reproduction. Typically, a host tissue is infected via an active invasion by the zoite form of parasites, which then divide intracellularly. Eventually, the cells burst, releasing zoites which infect new cells. This may occur several times, until gamonts are produced, forming gametes that fuse to create new cysts. There are many variations on this basic pattern, however, and many Apicomplexa have more than one host.

The Apicomplexa are an extremely large and diverse group, with well over 5000 named species. There are seven genera which infect humans: *Babesia, Cryptosporidium, Cyclospora, Isospora, Plasmodium, Sarcocystis* and *Toxoplasma.*

*Plasmodium,* as the causative agent of malaria, has the greatest impact on human health. Babesia is a relatively rare zoonotic infection. The other five species are all classified as coccidia.

Several apicomplexan parasites are also important in terms of veterinary medicine and agriculture. Most notable are *Babesia* and *Theileria* in cattle and *Eimeria* in poultry, cattle and sheep.

### VACCINE

The vaccine may be for treating and/or preventing a disease.

To "treat" means to administer the vaccine to a subject having an existing disease in order to lessen, reduce or improve at least one symptom associated with the disease and/or to slow down, reduce or block the progression of the disease.

To "prevent" means to administer the vaccine to a subject who has not yet contracted the disease and/or who is not showing any symptoms of the disease to prevent or impair the cause of the disease (e.g. infection) or to reduce or prevent development of at least one symptom associated with the disease.

The vaccine may comprise a plurality of amino acid/nucleic acid sequences corresponding to SEQ ID No. 1 homologues and/or SEQ ID No. 3 homologues from different strains and/or species of apicomplexan parasites.

Such "multi-strain" vaccines may comprise, for example, homologues from between two and ten strains of an apicomplexan parasite. The strains may, for example, represent those most commonly associated with pathogenesis in a particular territory or region, or they may be based on the strains associated with a recent (e.g in the last year, or 5 years) outbreak of the disease.

### DISEASE

Diseases caused by apicomplexan organisms include, but are not limited to Babesiosis (*Babesia* spp.) and Malaria (*Plasmodium* spp.), as well as the Coccidian diseases including: Coccidiosis (*Eimeria*.spp.); Cryptosporidiosis (*Cryptosporidium* spp.); Cyclosporiasis (*Cyclospora* spp.); Isosporiasis (*Isospora* spp.); and Toxoplasmosis (*Toxoplasma gondii).*

The disease coccidiosis may be caused by *Eimeria.*

Coccidiosis is one of the economically most important diseases in modern livestock production. For chickens the disease is caused by the replication within the intestine of the asexual and/or sexual stages of seven species of *Eimeria* (usually several species occur concurrently) and although overt clinical disease in intensively reared poultry is now relatively uncommon, sub-clinical infections are the norm. Sub-clinical infection can have a massive economic impact on livestock production, incurring reduced growth rates, poor feed conversion and a requirement for prophylactic control.

Coccidiosis is a parasitic disease that can cause severe losses in poultry meat and egg production. The parasites multiply in the intestines and cause tissue damage, lowered feed intake, poor absorption of nutrients from the feed, dehydration, and blood loss. Birds are also more likely to get sick from secondary bacterial infections. Eimerian infection can also predispose towards clinical disease caused by other pathogens, for example *Clostridium perfringens,* a cause of necrotic enteritis in chickens.

Chicken coccidia species include: *Eimeria acervulina; Eimeria brunetti; Eimeria maxima; Eimeria mitis; Eimeria necatrix; Eimeria praecox* and *Eimeria tenella.*

Turkey coccidia species include: *Eimeria adenoeides; Eimeria dispersa; Eimeria gallopavonis* and *Eimeria meleagrimitis..*

Bovine coccidia species include: *Eimeria alabamensis, Eimeria bovis* and *Eimeria zuernii.*

### ADMINISTRATION

The vaccine may be administered in a convenient manner such as by the oral, intravenous, intramuscular, subcutaneous, intranasal, intradermal or suppository routes or implanting (e.g. using slow release molecules).

For avian subjects, the vaccine may be administered individually to hatched chicks or chickens. Although accurate, these methods can be expensive e.g. for large broiler flocks. Alternative methods for treating the whole flock include *in ovo* vaccination, spray inoculation, provision of vaccine-containing gel blocks or administration to drinking water or feed.

Typically, a physician, veterinarian or producer will determine the actual dosage which will be most suitable for an individual subject or group of subjects and it may vary with the age, weight and response of the particular subject(s).

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as (or in addition to) the carrier, excipient or diluent, any suitable binder(s), lubricant(s), suspending agent(s), coating agent(s), solubilising agent(s), and other carrier agents that may aid or increase the delivery or immunogenicity of the vaccine.

For all vertebrate use, the vaccines may be administered parenterally, by injection, for example, either subcutaneously or intramuscularly. Additional formulations which are suitable for other modes of administration include suppositories and oral formulations. Where the vaccine composition is lyophilised, the lyophilised material may be reconstituted prior to administration, e.g. as a suspension.

Polypeptides may be formulated into the vaccine as neutral or salt forms. Pharmaceutically acceptable salts include the acid addition salts (formed with free amino groups of the polypeptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

### SUBJECT

The subject may be any organism susceptible to infection with an apicomplexan parasite. The subject may, for example, be a human.

The subject may be a livestock animal, such as a cow, sheep, goat or pig. The subject may be an avian subject, such as a bantam, chicken, duck, goose, guinea fowl, partridge, pheasant or turkey. The subject may be a farmed fish species.

Where the subject is an avian subject, the vaccine may be *administered in ovo,* to a chick (for example, a newly-hatched or one-day old chick) or to an adult bird.

The subject may be susceptible to apicomplexan infection.

Where the vaccine is used to treat an established infection, the subject may have been diagnosed as positive for the disease and/or show one or more symptom(s) associated with the infection.

### EXAMPLES

### Example 1 - Genetic characterisation of the E. maxima H and W strains

The *E. maxima* Houghton (H) and W strains are genetically and phenotypically distinct (Figure 2). The H strain is characterised by sensitivity to dietary robenidine at 66 ppm and complete escape from W strain-specific immune killing during passage in inbred Line C White Leghorn chickens induced by previous host exposure to the W strain. In contrast, the W strain was selected for resistance to 66 ppm robenidine but completely susceptible to W strain-specific immune killing. Genetic characterisation of the *E. maxima* H and W strains using amplified fragment length polymorphism (AFLP) with five different enzyme combinations to minimise restriction-associated bias generated a total of 3,230 genetic markers (see Table 1).

**Table 1 Frequency of five AFLP marker inheritance patterns. H parent specific negative selection = 66 ppm dietary robenidine. W parent specific negative selection = W strain-specific immunity induced by previous W strain infection. No significant difference was noted in the number of strain-specific markers amplified from either parental strain (Chi² test). No significant bias was detected in the identification of negatively selected markers between the enzyme combinations (Kruskal-Wallis test).**

| | AFLP enzyme combinations | | | | | | |
|---|---|---|---|---|---|---|---|
| Pattern | *Eco* RI/*Mse* I | *Taq* I/*Pst* I | *Bgl* II/*Mse* I | *Bgl* II/*Pst* I | *Eco* RI*lTaq* I | Total | % total |
| Common | 890 | 110 | 96 | 904 | 108 | 2108 | 65.3 |
| H parent specific, unselected | 371 | 16 | 24 | 164 | 11 | 586 | 18.1 |
| W parent specific, unselected | 289 | 13 | 22 | 155 | 19 | 498 | 15.4 |
| H parent specific, negatively selected | 2 | 0 | 0 | 0 | 0 | 2 | 0.1 |
| W parent specific, negatively selected | 19 | 0 | 0 | 15 | 2 | 36 | 1.1 |
| Total | 1550 | 139 | 142 | 1223 | 138 | 3230 | 100.00 |

Comparison between the two strains revealed 1,122 markers to be polymorphic (34.7%; as shown in Figure 2; a figure considerably higher than described previously for two *E. tenella* strains).

### Example 2 - Investigating the inheritance patterns of strain-specific (SS) markers

Inheritance patterns of these strain-specific (SS) markers by a parasite mapping panel (Table 2), which included the uncloned progeny of eight independent H/W strain crosses before and after concurrent immune/robenidine selection, revealed the absolute correlation of 36 and 2 markers with the immune and drug barriers respectively (Figure 2). Serial *in vivo* passage of four hybrid populations under double barrier selection for up to five generations did not change the marker inheritance profile.

**Table 2 The E. maxima parasite mapping panel. nr = not relevant.**

| Parasite population | Selection during passage | Generation(s) | Number of populations |
|---|---|---|---|
| H strain (parent) | None | nr | 1 |
| W strain (parent) | None | nr | 1 |
| Progeny of an H/W strain cross | None | 1 | 8 |
| Progeny of an H/W strain cross | W strain-specific immunity/robenidine | 1 | 8 |
| Progeny of an H/W strain cross | None | 2-3 | 4 |
| Progeny of an H/W strain cross | W strain-specific immunity/robenidine | 3-5 | 4 |
| Backcrossed progeny of an H/W strain cross | W strain-specific immunity/robenidine | 1-6 | 1 |

### Example 3 - Generation of hybridisation probes and identification of loci

Sequencing the 36 SS markers whose inheritance correlated with susceptibility to strain-specific immune selection identified 32 suitable for use as hybridisation probes. When radiolabeled with ³²P and used to probe an *E. maxima* W strain BAC library representing ∼7.5-fold genome coverage a series of BACs was identified. One of the identified BACs contained an AMA-1 homologue (BAC-1), a second BAC contained IMP-1 (BAC-2). Marker hybridisation to Southern blotted full PFGE-resolved karyotypes identified the chromosomal location of BAC-2 to be a band representing chromosomes 12, 13 or 14 (Table 3). These BACs were used in a protection trial (see figure legend for detail) and both conferred protection against challenge.

**Table 3 Summary of AMA-1 and IMP1 loci mapped within the E. maxima W strain genome associated with susceptibility to strain-specific immune killing.**

| Locus (antigen) | No. of marker s | No. BAC s | ∼ Mapped BAC size (Kb) | Chromosome | % genome* | % protection conferred** |
|---|---|---|---|---|---|---|
| 1 (AMA-1) | 3 | 1 | 127 | nd | 0.21 | **16**.**0±3**.**1^{a}** |
| 2 (IMP-1) | 2 | 1 | 90 | 12-14 | 0.15 | **59.7±6.8^{b}** |

| | | | | | | |
|---|---|---|---|---|---|---|
| *Based upon a predicted 60 Mb genome. **Immune protection associated with each mapped BAC was measured as the oocyst output resulting from W strain challenge following immunisation by infection with the heterologous H strain transiently transfected with one candidate BAC compared to transfection with a randomly selected control BAC. Comparison between the latter and unimmunised birds was not significantly different (5.4 ± 1.6). The average of three replicate transfections performed on separate occasions is shown. nd = not done. ^{a}Significantly different (p<0.01), ^{b}significantly different (p<0.005), using ANOVA + Tukey's post-hoc. | | | | | | |

### Example 4 - Fine mapping of protective antigens within the two loci

Confirmation of the potent immunogenicity of the early stages of the *E. maxima* lifecycle supported the development of an *in vivo* BAC-screening system to begin fine mapping for each locus (Table 4, trial 1). Utilising recent advances in transfection protocols for *Eimeria,* purified *E. maxima* H strain sporozoites were transiently transfected with each individual W strain BAC identified by hybridisation to an immune-correlated SS marker or a randomly selected control BAC. When Line C chickens were immunised by infection with 1 x 10⁶ test-, control- or non-BAC transiently transfected H strain sporozoites, drug-cleared by exposure to 66 ppm dietary robenidine and challenged three weeks later with the W strain the capacity to induce cross-protective immunity was seen to have been conferred by a single BAC from each mapped locus (Table 4, trial 2 and Table 3). A reproducible hierarchy was observed where a single BAC from both loci 1 and 2 consistently induced high levels of immune protection (Table 3).

**Table 4 Transient E. maxima BAC transfection: utility for in vivo immunisation and phenotypic screening for associated immunoprotective capacity. Immunisation included infection followed by drug clearance three days later using dietary robenidine (66 ppm). Challenge doses were administered three weeks after the immunising dose. Figures within each trial annotated with a different superscript letter were significantly different (p<0.05; ANOVA + Tukey's post hoc). ND = none detected. *Control (randomly selected) BAC = EmaxBAC4c21, **Test BAC = EmaxBAC2k08 (BAC-2), representing immune mapped locus 2.**

| Trial | Immunisation | | Electroporation | Challenge | Oocysts excreted per bird ± SEM (x10⁶) |
|---|---|---|---|---|---|
| | Vehicle | BAC | | | |
| 1 | None | None | No | *E. maxima* H | 19.4 ± 1.8^{a} |
| | *E. maxima* H | None | No | *E. maxima* H | ND |
| 2 | None | None | No | *E. maxima* W | 25.8 ± 2.3^{a} |
| | *E. maxima* H | None | Yes | *E. maxima* W | 22.0 ± 2.6^{a} |
| | *E. maxima* H | Control* | Yes | *E. maxima* W | 24.4 ± 2.7^{a} |
| | None | Test** | Yes | *E. maxima* W | 24.6 ± 2.0^{a} |
| | *E. maxima* H | Test* | No | *E. maxima* W | 21.0 ± 3.9^{a} |
| | *E. maxima* H | Test** | Yes | *E. maxima* W | 8.1 ± 0.7^{b} |

The parasite mapping panel was supplemented by backcrossing and re-selecting one double barrier-selected population to the immune-targeted parental W strain on six successive occasions. Genotyping each selected backcross generation revealed the reappearance of 21/32 hybridisation SS markers, between one and three SS markers remaining absent for all mapped loci (e.g. Figure 3a). Backcross genotyping fine-mapped locus 1 to ∼64 Kb genomic sequence contained within BAC *Emax*BAC8f18. Typing additional SS PCR markers produced by targeted sequencing from the H strain across locus 1 provided further focus, mapping to ∼50 Kb (Figure 3b, Tables 5 and 6).

**Table 5 Primers for PCR amplification of equivalent H strain regions to identify polymorphic markers across locus 1 (BAC EmaxBAC8f18).**

| Genetic marker | Forward primer | Reverse primer |
|---|---|---|
| EmBAC8f18_01w | 5'-ttgagttgggcgattttccttt-3' | 5'-acgaggacacgtagctggtctg-3' |
| EmBAC8f18_02w | 5'-cgcgtgtgtggtacaaacaaaa-3' | 5'-agtccatggcactccacacaat-3' |
| EmBAC8f18_03w | 5'-taccgagcaacaaggcaggtaa-3' | 5'-tcccatattttgccatttgctg-3' |
| EmBAC8f18_04w | 5'-ctggtggacgatgttctggttc-3' | 5'-gtctattcaacaccggcagtcg-3' |
| EmBAC8f18_05w | 5'-ggatcacattccgttcaactcg-3' | 5'-tcgacagctgcaaaagaaaagc-3' |
| EmBAC8f18_06w | 5 '-cttctgatctcccgctttcaca-3' | 5'-tcttgccgttgttctcgtcttc-3' |
| EmBAC8f18_07w | 5'-ttccctttttgcgaatctccat-3' | 5'-cctctggctgaaagaagcaaca-3' |
| EmBAC8f18_8w | 5'-gtcaggccaataacagccacag-3' | 5'-ccccatccatccttagaagtgc-3' |
| EmBAC8f18_09w | 5'-tttcctccttttccccctcttc-3' | 5'-accgaaggaaaagggtttaggg-3' |
| EmBAC8f18_10w | 5'-cctccctccctctttttccttg-3' | 5'-agcagcagcaggaagagtgtgt-3' |
| EmBAC8f18_11w | 5'-gggagaggaaggggagactgat-3' | 5'-tcctcgattctacctgctgctg-3' |
| EmBAC8f18_12w | 5'-agcaagcaatgacaagcacctc-3' | 5'-gtggatgggtggggattatagg-3' |

**Table 6 W strain-specific primers for genotyping backcrossed populations (derived using sequences from Table 5). Upper case letters are polymorphic between the E. maxima H and W strains.**

| Genetic marker | Forward primer | Reverse primer |
|---|---|---|
| EmBAC8f18_01w | 5'-tgagacgggaactttgcttctG-3' | 5'-tagcatcgagaaatcgccgttA-3' |
| EmBAC8f18_02w | 5'-cttggctcaccaatgtcacctC-3' | 5'-accttcctttaccattccgacT-3' |
| EmBAC8f18_03w | 5'-agtctcctttggtacccggtgA-3' | 5'-cttgcgtatccatgatagctcT-3' |
| EmBAC8f18_04w | 5'-gtgggtaagcgctttggagagT-3' | 5'-tgggctctacagcagctgaaaC-3' |
| EmBAC8f18_05w | 5'-tgtcatcgacaaggaacatctcT-3' | 5'-gtgcttctttcggaggcatctT-3' |
| EmBAC8f18_06w | 5'-ttctgaaatggggtgtatagacG-3' | 5'-ctaaaacctcttccctgggC-3' |
| EmBAC8f18_07w | 5'-aaaagattagttgaatatctgaggaGA-3' | 5'-gaagcaacaagcgcatctaatC-3' |
| EmBAC8f18_08w | 5'-agagggaaaatcaatgcaagaC-3' | 5'-gcagcagcaGCTgctgCT-3' |
| EmBAC8f18_09w | 5'-gtgtgcgtgataaggagccC-3' | 5'-atctaaaagtaagtcttttcttcttttttTC-3' |
| EmBAC8f18_10w | 5'-gcttactctgtatagtaccatccttattT-3' | 5'-ttattttttctcctgctGctC-3' |
| EmBAC8f18_11w | 5'-gatggatccgtaggtggtgttG-3' | 5'-tgctgcagtgtctgtttgcttC-3' |
| EmBAC8f18_12w | 5'-ctcagggcagTctagtcttcC-3' | 5'-gattgcattcagtcgtgacagC-3' |

Examination of fine-mapped locus 1 identified three predicted coding regions, annotated as encoding (i) a sulphate transporter, (ii) an apical membrane antigen-1 (AMA-1) homologue and (iii) a transcription elongation factor (Figure 3c). Sequencing from the H strain identified likely coding polymorphism in the first two candidates but not the third. *In vivo* infection by H strain sporozoites transiently transfected with purified genomic LD PCR amplicons covering each predicted coding sequence and flanking regions induced a cross-protective immune phenotype only when using *EmAMA-1* (Figure 3d). Immunisation using EmAMA-1 as a DNA vaccine in the eukaryotic expression vector pcDNA3.1(+) (Invitrogen) or as a bacterially-expressed recombinant protein induced significant immune protection against subsequent challenge by the W strain (Figure 3d). Comparison between the H and W *EmAMA-1* coding sequences reveal four nucleotide polymorphisms, two of which yield non-synonymous changes, one in the likely pro-domain and one in domain 1. AMA-1 domain 1 is known to be highly polymorphic among *Plasmodium falciparum* strains and this polymorphism confers the highest level of escape from inhibitory antibodies.

Whole BAC transient transfection identified locus 2, contained within BAC *Emax*BAC2k08, as capable of inducing the most protective immune response (Table 3, Figure 4a). Backcross genotyping failed to provide any further focus, yielding a region nearly double the size of that found for locus 1 (Figure 4b). The difference in locus size may reflect polymorphism in genome-wide recombination rates, as have been reported for other Apicomplexa. Targeted disruption at eight predicted coding regions identified by homology to other annotated genes or EST sequences and clusters of candidate open reading frames created a panel of eight otherwise unaltered *Emax*BAC2k08 versions (Figure 4c and Table 7).

**Table 7 BAC recombineering constructs created by PCR amplifying a selectable cassette (β-lactamase, using pGEM-Teasy as template, primer sequences A) incorporating unique BAC sequences for targeted recombination (sequences B).**

| |
|---|
| Forward B A β -lactamsase CDS A B Reverse |

| **A: selectable cassette specific primer sequences** |
|---|
| Forward: 5'-agagttggtagctcttgatc-3' |
| Reverse: 5'-cattcaaatatgtatccgctc-3' |

| **B: BAC target specific primer sequences (recombination sites)** |
|---|
| Target 1 forward: 5'-cgatttaccctcagcaaatgatagcaacacgttaggtatacgtgctcgtt-3' |
| Target 1 reverse: 5'-tctatggatactgcgctgcagcagcagcgcgggcatacttagttgctgca-3' |
| Target 2 forward: 5'-cagtgtatctagtgtatactgaggtgcctatacaccgcaaatagtaaata-3' |
| Target 2 reverse: 5'-tacctgcctatacaccccaatattgacgaagtgaccactggtcagtttat-3' |
| Target 3 forward: 5'-tgggagcggtggcagtgtaagtacaccgctgcaccatcagtagctgctgc-3' |
| Target 3 reverse: 5'-tgtcttaactgcaacagatgtgcgcatgccaagactggcgcctgaattac-3' |
| Target 4 forward: 5'-taatatgggtgcccgttgtctgccaaagatttggcagtgcagcatggaac-3' |
| Target 4 reverse: 5'-aacaaaatcgtcaatcccaagtacaacaacggacatcctgtctttcggaa-3' |
| Target 5 forward: 5'-tacgaacaatgaggacagatattgacgcggttttcttcaggagaaggaat-3' |
| Target 5 reverse: 5'-ccaatggaccaagccatccatgcacgcgacaggagcattcgagtacctgt-3' |
| Target 6 forward: 5'-ctgtagtacaatgacaccttcatctcctgaaacatcaaacatacgacaaa-3' |
| Target 6 reverse: 5'-tggggcctcccccaaaacttacacaatggagcagatacgtttactgtccg-3' |
| Target 7 forward: 5'-ttcttctacgcgcaggcagacaggcggacccaactggtacacactcgtga-3' |
| Target 7 reverse: 5'-tgtgagggtgggttctcccgcgtaggctcatgcagctgttcatactggtc-3' |
| Target 8 forward: 5'-gaatgccattcccttgccaccgattgactaaagtctacgtgaattctgca-3' |
| Target 8 reverse: 5'-caaataaattcctcaataataatcaatagtcgtcttatttagatgcatgc-3' |

Comparison of cross-protective immunity induced following infection by H strain sporozoites transiently transfected with the unaltered *Emax*BAC2k08 or a deletion mutant daughter BAC revealed reduced protection across three disrupted regions, most notably region 7 (Figure 4d). *Not* I/*Sfi* I digestion of *Emax*BAC2k08 yielded ∼63.7 and ∼22.4 Kb fragments of the insert separate from the vector, the smaller fragment representing the three candidate disrupted regions. Immunisation using H strain sporozoites transiently transfected with either BAC fragment confirmed the induction of a cross-protective immune phenotype associated with the smaller but not the larger fragment (40% and -16% protection respectively compared to a randomly selected BAC control). Homology-led gene annotation suggested the presence of two coding sequences within the fine mapped ∼22.4 Kb locus. More detailed scrutiny using a locus-wide NimbleGen tiling array with cDNA derived from *E. maxima* W strain sporozoites, merozoites (harvested 67 hours post infection, hpi) and chicken intestine centred on Meckel's diverticulum without infection or 6 and 16 hpi with 1 x 10⁶ W strain oocysts, revealed four sequences transcribed by the stages tested (Figure 4e-g). Although eimerian lifecycles are relatively complex, the choice of lifecycle stages to be sampled was informed by quantitative PCR-based enumeration of *E. maxima* replication in naive and immunised Line C chickens, which revealed the first 24 hpi to cover the period of most immune killing during homologous challenge (∼88% reduction at 20 hpi; Figure 5).

Examination of the array-identified transcribed sequences revealed (i) a putative non-coding RNA, (ii) an unknown coding sequence temporarily termed *IMP-1,* (iii) a putative cyclophilin-RNA interacting protein and (iv) a SCY kinase-related protein. Infection by H strain sporozoites transiently transfected with LD PCR amplicons covering each predicted W strain coding sequence and flanking regions, including a section of repeats not found to be transcribed but identified as a cross-reactive feature by the array, induced a cross-protective immune phenotype only when using *EmIMP-1* (Figure 4h). Subsequently, immunisation of Line C chickens using EmIMP-1 as a bacterially-expressed recombinant protein induced 45% immune protection against challenge by the W strain (compared to the thioredoxin protein control, 50% compared to the unimmunised control; Figure 6). Comparison between the H and W *EmIMP-1* coding sequences uncovered five nucleotide polymorphisms, two of which yield non-synonymous changes.

Further characterisation of EmIMP1 has identified potential homologues within the genomes of other coccidial parasites including *E. tenella* (2e-87; NCBI BLASTp2seq), *T. gondii* (XM_002370108; 2e-36) and *Neospora caninum* (GeneDB NCLIV_000430; 4e-37), all of which share a common intron/exon structure (Figure 1).

The identification of new antigens as highly protective anti-coccidial vaccine candidates is a significant step towards a new generation of cost-effective vaccines. Such vaccines have the potential to revolutionise poultry production and welfare, as well as impacting on the lives of many of the poorest people in the world.

### MATERIALS AND METHODS

**Parasites and animals.** The *E. maxima* Houghton (H, sensitive to dietary robenidine) and Weybridge (W, resistant to 66 ppm dietary robenidine) strains were used as the parental parasites in these studies. Oocysts were propagated and genetic crosses were carried out *in vivo* as described previously (Blake et al. (2004) Mol Biochem Parasitol 138: 143-152).

**Experimental design: genetic crosses and selection of progeny populations.** Uncloned populations of hybrid parasites were derived from eight independent crosses between the W and H *E. maxima* strains in Line C White Leghorn chickens as described previously (Blake et al. (2004) as above; oocysts recovered from two-ten birds and pooled for each population). Hybrid sub-populations were derived from each cross following *in vivo* passage under a double selective barrier comprising W strain-specific immune (induced by previous infection with 100 oocysts of the pure W strain) and H strain-specific drug (66 ppm robenidine) selection (Blake et al. (2004) as above). All eight first generation selected parasite populations, together with four serially-selected populations (three to five rounds of selection, two-ten birds pooled per population per generation), were used to prepare the parasite mapping panel (Table 2).

One selected parasite population was backcrossed six times under double barrier selection, each backcross generation derived *after in vivo* phases of (i) cross fertilisation: *in vivo* passage using 100 sporulated hybrid oocysts with 400 sporulated W (immune-targeted) parental strain oocysts and (ii) selection: passage of 10,000 sporulated recovered parasites under double barrier selection. Parasites recovered from ten birds were pooled for each backcross stage.

**Experimental design: *in vivo* protection trials.** Experiments to measure immune protection induced by (i) immunisation through previous parasite exposure, (ii) recombinant protein or (iii) DNA vaccination followed standardised protocols. All treatment groups comprised six individually caged specific pathogen free Line C White Leghorn chickens. Total daily oocyst excretion per bird was determined following daily faecal collection from days 6-7, 7-8 and 8-9 post infection by flotation in saturated salt solution using a modified McMaster protocol as described previously (Blake et al. (2004) as above). All experiments included a non-immunised control group.
(i) Previous parasite exposure. Three week old chickens were immunised by oral infection with 100 sporulated *E. maxima* oocysts (wild type) or 1.0 x 10⁶ transfected *E. maxima* sporozoites (following oral gavage with 0.5 ml 5% w/v sodium bicarbonate solution; drug cleared three days post immunisation by inclusion of dietary robenidine at 66 ppm for three days). Six week old chickens were challenged with 250 sporulated *E. maxima* oocysts.
(ii) Recombinant protein. Two week old chickens were immunised subcutaneously using 100 µg PBS-dialysed recombinant protein in TiterMax Gold adjuvant (Sigma-Aldrich Ltd.) at two sites in the neck. Chickens were subsequently re-immunised at three and four weeks of age (as before but in Freund's Incomplete adjuvant; Sigma-Aldrich Ltd.) prior to challenge with 250 sporulated *E. maxima* oocysts when six weeks old. Unimmunised, PBS alone and PBS-dialysed thioredoxin were included as negative control groups.
(iii) DNA. Two week old chickens were immunised intramuscularly using 50 µg DNA vaccine plasmid in sterile endotoxin-free TE buffer (Qiagen) at two sites in the thigh. Chickens were subsequently re-immunised at three and four weeks of age (as before) in alternating legs prior to challenge with 250 sporulated *E. maxima* oocysts when six weeks old. TE alone and vector pcDNA3.1(+) (Invitrogen) were included as negative controls in addition to an unimmunised group. Tissue excised from the immunisation site post-mortem from one extra bird per group seven days post-immunisation was processed to extract total RNA using the Qiagen RNeasy mini kit (Qiagen) for cDNA preparation and PCR to confirm DNA vaccine transcription (data not shown).

**Nucleic acid resources.** Genomic DNA was extracted from oocysts as described previously using a physical smashing step followed by phenol/chloroform extraction (Blake et al (2003) Parasitology Research 90: 473-475) and from chicken intestinal tissue samples using a Qiagen DNeasy tissue kit as described by the manufacturer (Qiagen) followed by RNase A treatment (Blake et al (2006) Int J Parasitol 36: 97-105). Total RNA was purified from *E. maxima* sporozoite, merozoite (67 hours post infection), infected and uninfected chicken intestinal tissue using a Qiagen RNeasy kit as described by the manufacturer. A BAC library was constructed for the *E. maxima* W strain in the pBACe3.6 vector following the protocol of Osoegawa et al (Osoegawa et al. (1998) Genomics 52: 1-8) based upon chromosomal DNA prepared from *E. maxima* sporozoites as described elsewhere (Shirley et al (1990) Mol Biochem Parasitol 38: 169-173). BAC DNA was prepared using the Qiagen Large-Construct kit as described by the manufacturer (Qiagen).

Standard PCR amplification was completed using BIO-X-ACT Short DNA Polymerase (Bioline Ltd.). Each PCR reaction contained 5 ng template DNA, 20 pmol of relevant forward and reverse primers, 0.5 U *Taq* polymerase, 10 mM Tris-HCl, 1.5 mM MgCl2, 50 mM KCl and 0.2 mM dNTPs. Standard cycle parameters were 1 x (5 min at 94 °C), 30 x (1 min at 94 °C, 1 min at 54-58°C and 1-5 min at 72 °C) and 1 x (10 min at 72 °C). For LD PCR BIO-X-ACT Long DNA Polymerase (Bioline Ltd.) was used as recommended by the manufacturer. Where required cDNA was prepared using Invitrogen Superscript II reverse transcriptase and oligo dT as described by the manufacturer (Invitrogen Ltd.). PCR fragments were cloned using pGEM^{®}-T Easy (Promega) in XL1-Blue *Escherichia coli* (Stratagene), miniprepped (Qiagen) and sequenced (Beckman CEQ 8000 genetic analysis system) as described by the respective manufacturers. Sequence assembly, annotation and interrogation were undertaken using VectorNTI v11.0 (Invitrogen) except where stated.

**Quantification of parasite replication.** Groups of four inbred SPF Line C White Leghorn chickens were either left naive or were immunised by infection with 100 *E. maxima* W strain oocysts at three weeks of age. All birds were subsequently challenged by infection with 1.0 x 10⁶ *E. maxima* W strain oocysts at six weeks of age (homologous challenge). Unimmunised and immunised groups were culled at 0, 2, 4, 6, 8, 12, 16, 20, 24, 32, 40, 48 and 72 hours post challenge, when an 8 cm length of intestine centred on Meckel's diverticulum was recovered post-mortem from each test bird and homogenised in sterile TE using a Qiagen TissueRuptor (230 V, 50/60 Hz). Total genomic DNA was extracted from three 25 µl aliquots of each sample using a Qiagen AllPrep DNA/RNA Mini kit as described by the manufacturer (Qiagen). The total numbers of *E. maxima* genomes per host genome were determined from each sample using TaqMan quantitative PCR assays specific for the *E. maxima* microneme protein 1 (MIC1) and chicken glyceraldehyde 3-phosphate dehydrogenase (GAPDH) loci in duplex with the 7500 Fast Real-Time PCR System (Applied Biosystems) (Blake et al (2006) as above). TaqMan probes were 5' labeled with FAM (MIC1) or Yakima Yellow (GAPDH) and 3' quenched with Eclipse Dark Quencher (Eurogentec). TaqMan conditions and cycle parameters were modified from the standard Applied Biosystems Fast protocol (40 cycles: 1 x 95 °C, 20 s; 40 x 95 °C, 15 s and 60 °C, 30 s). Quantitative calculations were facilitated and validated by comparison with known concentrations of the relevant genomic DNA template.

**Genetic marker production.** AFLP was used to generate the majority of the genetic markers used during these studies as described elsewhere (Vos et al. (1995) Nucleic Acids Research 23: 4407-4414). Approximately 50 ng total *E. maxima* genomic DNA was digested using one of five restriction enzyme combinations (Table 1; New England Biolabs) prior to ligation to adapters derived from those described by Vos et al, adapted for the respective restriction enzyme (Vos et al (1005) as above). Primer pairs (MWG Biotech (UK) Ltd.) were based on the adaptor sequences and provided 0 and 1 (primary amplification) or 1 and 2 (secondary amplification) selective bases, respectively. Markers of interest were gel excised, re-amplified, cloned and sequenced as described previously (Blake et al (2004) as above. Marker specific-primers were designed using Primer3 to amplify marker-associated DNA fragments.

Additional genetic markers were developed by sequencing 600-750 bp sections of *E. maxima* H strain genomic DNA corresponding to targets distributed across each locus mapped in the W strain (Table 5 for the primers used). Strain-specific primer pairs were developed following sequence alignment and SNP identification (ClustalX; Table 6).

**Pulsed field gel electrophoresis.** Chromosomal karyotypes were resolved by PFGE. Briefly, *E. maxima* W strain chromosomal DNA in ∼4 mm x 3 mm x 2 mm sections cut from agarose blocks were separated in 0.8% SeaKem HGT agarose gels (Lonza) prepared in 0.5 x Tris-borate EDTA (TBE) buffer and subjected to PFGE in a 21 cm x 14 cm gel using a CHEF DRII system (Bio-Rad) in 2 L of 0.5 x TBE running buffer at 14 °C. PFGE conditions were (i) 216 h at 1.3 volts/cm with a switch time of 3,000 - 3,500 s followed by (ii) 120 h at 1.3 volts/cm with a switch time of 3,300 - 3,600 s finishing with (iii) 48 h at 1.2 volts/cm with a switch time of 3,200 - 3,400 s. Gels were stained in a 0.5 µg/ml aqueous solution of ethidium bromide for 30 min, destained in water and then photographed. *Hansenula wingei* and *Schizosaccharomyces pombe* DNA plugs (Bio-Rad) were included as size markers.

Individual BAC clone insert size was determined by *Not* I (New England Biolabs) digestion followed by PFGE in 1% Bio-Rad pulsed field certified agarose prepared in 0.5 x TBE as above. PFGE conditions were 18 h at 4 volts/cm with a switch time of 1 - 6 s. Low range PFG size markers (New England BioLabs) were included as size markers.

**DNA hybridisation.** PFGE-resolved chromosomal DNA was transferred to Hybond-N+ membrane (Amersham Biosciences) as recommended by the manufacturers. 3072 BAC transformed *E. coli* DH10B were robotically gridded onto replicated filter arrays providing ∼7.5-fold coverage of the *E. maxima* W strain genome. PCR products derived from AFLP markers of interest were labelled with ³²P using a Prime-It II random priming kit (Stratagene) and hybridised to filters for 16-24 h at 65 °C as described by Amersham Biosciences. Filters were washed three times at 65 °C in 0.1 x SSC before exposure to X-ray film (Kodak BioMax MS) at -80 °C against intensifying screens.

**BAC sequencing, assembly and annotation.** BAC DNA was prepared from clones identified by hybridisation to AFLP markers of interest using the Qiagen Large-Construct kit as recommended by the manufacturer. Small insert libraries (2-4 Kb) were prepared by shearing the DNA by sonication, blunt ending and size selecting by agarose gel electrophoresis prior to sub-cloning into *Sma* I digested dephosphorylated pUC18 for use in a whole-BAC shotgun sequencing strategy. ABI PRISM BigDye Terminator (Applied Biosystems) forward and reverse plasmid end sequences generated using an ABI3730 capillary sequencer were assembled using the Staden-based PHRAP (P. Green, unpublished). Contig assembly was based upon LD PCR.

Preliminary annotation of each assembled BAC sequence was achieved using tBLASTx interrogation of all publically accessible sequences through the National Center for Biotechnology Information (NCBI, http://www.ncbi.nlm.nih.gov/), supplemented by *Eimeria* species EST data from the *Eimeria* ORESTES and *E*. *maxima* EST sequencing projects (Gruber and Madeira, unpublished; Wan and Blake, unpublished, respectively).

**BAC recombineering.** The *E. coli* DY380 strain (kindly supplied by the National Cancer Institute, Frederick, USA) was initially transformed with *E. maxima* W strain BAC *Emax*2K8 as described elsewhere (Thomason et al. (2007) Curr Protoc Mol Biol Chapter 1: Unit 1 16). Subsequently, the DY380/*Emax*BAC2K8 line was transformed with each of eight PCR products representing (i) unique *Emax*BAC2K8 sequences for targeted homologous recombination and (ii) the β-lactamase coding sequence amplified from pGEM Teasy (Promega; primers as shown in Table 7; PCR as above, purified using the Qiagen Gel Extraction kit as described by the manufacturer)^{[27]}. The resulting bacterial strains were sub-cloned and tested for evidence of correctly targeted insertion in a pure clonal line and the absence of widespread BAC disruption by (i) positive PCR between insert and flanking BAC sequences (Figure 7), (ii) negative PCR between target and flanking BAC sequences (Figure 7, primers shown in Table 8) and (iii) unchanged BAC PFGE profile following *Not* I/*Sfi* I digestion (Figure 8; enzymes New England Biolabs).

**Table 8 Validation of targeted BAC recombineering by PCR. Primers designed to confirm targeted disruption: knockout = amplification between target-flanking genomic DNA and the insert, wild-type = amplification between target-flanking genomic DNA and the genomic DNA target. Results shown in Figure 7.**

| Target | Insertion result | Forward primer | Reverse primer |
|---|---|---|---|
| 1 | Knockout | 5'-cagcagcgta acttggaggaga-3' | 5'-agcgtcagacc ccgtagaaaag-3' |
| | Wild-type | As for Target 1 positive | 5'-gctgcggagt cagtgtagctgt-3' |
| 2 | Knockout | 5'-atcccttctc gtttgctgcatt-3' | As for Target 1 positive |
| | Wild-type | As for Target 2 positive | 5'-gctgctgctgc taaggagactg-3' |
| 3 | Knockout | 5'-gggccccaata gataaggaggt-3' | As for Target 1 positive |
| | Wild-type | As for Target 3 positive | 5'-ggtagtggcgc agttattggtg-3' |
| 4 | Knockout | 5'-atgagcatagg atgcaggcgta-3' | As for Target 1 positive |
| | Wild-type | As for Target 4 positive | 5'-cttggagttct tgccgatcctt-3' |
| 5 | Knockout | 5'-ccgaccgaagt tttgttcacac-3' | As for Target 1 positive |
| | Wild-type | As for Target 5 positive | 5'-gcgggtacaag cagtagaagca-3' |
| 6 | Knockout | 5'-gaggcggtttc ctttatgatgc-3' | As for Target 1 positive |
| | Wild-type | As for Target 6 positive | 5'-gtgcttcaaagt cgctgctcac-3' |
| 7 | Knockout | 5'-gtggagctttt cctggtggagt-3' | As for Target 1 positive |
| | Wild-type | As for Target 7 positive | 5'-cgttttcaggtgg ggcttacat-3' |
| 8 | Knockout | 5'-ataagggcgaca cggaaatgtt-3' | 5'-tgcaagcatg tgcatccaata-3' |
| | Wild-type | 5'-gtggtcgcatatc tgcatcagg-3' | As for Target 7 positive |

**Transfection.** *E. maxima* W strain genomic DNA, presented as whole, recombineered or partial BAC-encoded templates or LD PCR amplicons, was used to transiently transfect the *E. maxima* H strain. For whole and recombineered BAC transfection purified plasmid DNA was re-suspended at 50 µg/10 µl TE. BAC *Emax*BAC2k08 was subdivided by *Not* I/*Sfi* I digestion (New England Biolabs) and subsequent PFGE (as above), yielding ∼63.7 and ∼22.4 Kb fragments of the insert as well as the vector. The 63.7 and 22.4 Kb fragments were gel excised following large-scale PFGE, electroeluted into dialysis bags in TE, precipitated and re-suspended at 20 µg/10 µl in TE. LD PCR amplicons representing three *Emax*BAC8f18 candidate regions and four *Emax*BAC2k08 regions were amplified in triplicate, (primers shown in Table 9, designed to lie >1 Kb outside any hit with an E value of 1e-05 or below or to yield an amplicon > 7 Kb in size across the predicted coding sequence, whichever was the greater). All triplicates were electrophoresed to check for purity and target size, identified by test secondary PCR (Table 9), pooled once validated, precipitated and re-suspended in 10 µl TE.

**Table 9 EmaxBAC8f18 and EmaxBAC2k08 specific primers for LD PCR and nested PCR to confirm LD PCR amplicon identity. *Putative annotation.**

| BAC ID | Target | LD PCR primers | Nested confirmatory primer |
|---|---|---|---|
| *Emax*BAC8 f18 (see **Figure 3****)** | Sulphate transporter* | For: 5'-gggcaacag cagtctcacatct-3' | For: 5'-tgtggtattgt aggcggattgg-3' |
| | | Rev: 5'-caagttgca cccgagtcagtct-3' | Rev: 5'-tgcctgagg accttgcaataaa-3' |
| | Apical membrane antigen 1 | For: 5'-ggctttcgtg gatctacctgct-3' | For: 5'-gttcatggac ggggtttgtgta-3' |
| | | Rev: 5'-gtctgcaca cctcaacagcaga-3' | Rev: 5'-ggtcggcta tctagcgctttgt-3' |
| | Transcription elongation factor* | For: 5'-cgaacaca gtctccttgccagt-3' | For: 5'-ccgtctgtgt accagcctctgt-3' |
| | | Rev: 5'-tcctcgatt ctacctgctgctg-3' | Rev: 5'-caattcttca accttgcgaacg-3' |
| *Emax*BAC2 k08 (see **Figure** 4) | ncRNA | For:5'-aagagggag gagggggtacaga-3' | For: 5'-caaacccgt aagcccggtaata-3' |
| | | Rev: 5'-ttcgtcaaag cagcagatgaga-3' | Rev: 5'-tgtttgtttgc tcaccgtctga-3' |
| | Unknown | For: 5'-taattgccgc accatacaggtt-3' | For: 5'-tcgccgaatc tctttggtgtaa-3' |
| | | Rev: 5'-gagaggga gaggtcggtgtgtt-3' | Rev: 5'-tacaagtggga ggtttggagca-3' |
| | Repeats + Cyclophilin-RNA interacting protein* | For: 5'-agcaacagca gccatacaatcc-3' | For: 5'-tgctgctgctt catcctcttct-3' |
| | | Rev: 5'-atgggcacaa gaaaaagggaaa-3' | Rev: 5'-gtggtggtgg ggttgttgtatg-3' |
| | SCY kinase related protein* | For: 5'-gcacatgcttg caaaccctaaa-3' | For: 5'-atgagtgcaat gctgtccgatt-3' |
| | | Rev: 5'-aagctcgggt gtctgtgtcaac-3' | Rev: 5'-tgctctgtctgc acgattgaag-3' |

Transient transfection was accomplished following a protocol modified slightly from that described previously (Clark et al. (2008) Mol Biochem Parasitol 162: 77-86). Briefly, freshly excysted and purified *E. maxima* H strain sporozoites were washed in incomplete cytomix and re-suspended in AMAXA Basic Parasite Nucleofector Solution 2 at 3.0 x 10⁷/ml immediately prior to nucleofection. Subsequently, 100 µl sporozoite suspension was mixed with 10 µl DNA at room temperature, transferred to a cuvette and nucleofected as described by the manufacturer using a Nucleofector II with program U-033 (Lonza). Post-nucleofection all sporozoites were immediately re-suspended in 3 ml PBS + 1% glucose (w/v) and left to rest for 20 mins at room temperature. Sporozoites were then either used as an oral dose to initiate *in vivo* infection or incubated overnight at 41 °C in a 5% CO₂ incubator. For oral dosing the output from two nucleofections were pooled and dosed using 1.0 x 10⁶ sporozoites (counted pre-nucleofection) per bird. Total RNA was extracted from incubated sporozoites using the Qiagen RNeasy mini kit for cDNA preparation and PCR of one or more transfected DNA-specific transgenes to confirm transfection (data not shown).

**BAC tiling array.** A custom-made NimbleGen genome tiling array containing 79,955 50-75 bp probes (presented as forward and reverse strands in duplicate) was designed and produced using BAC sequences obtained during these studies (Roche). *E. maxima* W strain genomic DNA covered by BACs *Emax*BAC8f18 and *Emax*BAC2k08 were represented by 9,798 and 7,387 probes respectively (*Emax*BAC8f18: 8,321 unique, 1,477 twice, 84.5% coverage; *Emax*BAC2k08: 7,315 unique, 72 twice, 95.5% coverage). Total RNA extracted from purified *E*. *maxima* W strain sporozoites, merozoites (harvested 67 hpi), uninfected chicken intestinal tissue sampled at Meckel's diverticulum and chicken intestinal tissue sampled 6 and 16 hpi by 1 x 10⁶ *E. maxima* W strain oocysts were processed to produce labelled cDNA and hybridised to the array as described by the manufacturer (Roche). Arrays were scanned using a GenePix 4000B scanner and the images were visualised using GenePix Pro software (Axon). Array design was viewed using NimbleGen SignalMap v1.9 software. Arrays were read using NimbleGen NimbleScan v2.5 software with alignment and uniformity cut off points of 0.15 and 0.3 respectively.

**Recombinant protein vaccine preparation.** cDNA sequences corresponding to the predicted AMA-1 ectodomain and IMP-1 proteins (loci 1 and 2 respectively: AMA-1 coding nucleotides 79-1,347, IMP-1 161-1,275) were amplified from *E. maxima* W strain sporozoite cDNA, cloned into the expression vector pET32b (Novagen) using *Bam* HI/*Hind* III and *Nco* I/*Eco* RI restriction sites respectively and sub-cloned in *E. coli* BL21(DE3)pLysS (Novagen). Recombinant proteins were expressed and purified using HisTrap FF purification columns (GE Healthcare) as described by the respective manufacturers, dialysed overnight against PBS and finally mixed with an equal volume of adjuvant shortly before use. Thioredoxin expressed in the same manner using the unmodified pET32b vector was purified and used as a negative control.

**DNA vaccine preparation.** cDNA sequences corresponding to the predicted AMA-1 ectodomain and sulphate transporter proteins (locus 1: AMA-1 coding nucleotides 79-1,347, sulphate transporter 1-2,988) were amplified from *E*. *maxima* W strain cDNA, cloned into the eukaryotic expression vector cDNA3.1(+) (Invitrogen) using *Hind* III/*Bam* HI restriction sites and sub-cloned in *E. coli* XL1-Blue MRF (Stratagene). Plasmid DNA was purified using the Qiagen Plasmid Maxi kit as recommended by the manufacturer (Qiagen), precipitated and re-suspended in TE at 250 µg/ml.

**Statistics.** Arithmetic mean and associated standard error of the mean (SEM) for each sample or group were calculated using Excel (Microsoft Excel 2002, Microsoft Corporation, 2001). Statistical analyses were performed using the t-test, ANOVA, Chi² or Kruskal-Wallis tests in Minitab (Minitab Release 14, Minitab Inc., 2003), complimented by post hoc analysis using the Tukey's test. Differences were deemed significant with a *p* value < 0.05.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A polypeptide from an *Eimeria* species parasite comprising the amino acid sequence as shown as SEQ ID No. 1 or the *Eimeria tenella* amino acid sequence shown as Et IMP-1 shown in Figure 1 (b) or a variant having at least 80% sequence identity to SEQ ID NO: 1 and being capable of inducing a protective immune response in a subject against subsequent challenge with an apicomplexan parasite.

2. A nucleic acid capable of encoding a polypeptide according to any preceding claim.

3. A nucleic acid sequence according to claim 2, comprising the coding part of the nucleotide sequence shown as SEQ ID No. 2 or 6.

4. A vector comprising a nucleic acid according to claim 2 or 3.

5. A vaccine for the treatment and/or prevention of a disease associated with an *Eimeria* species parasite, which comprises:
(i) a polypeptide according to claim 1; and/or
(ii) a nucleic acid sequence capable of encoding such a polypeptide.

6. A vaccine according to claim 5, wherein the disease is coccidiosis.

7. A vaccine according to claim 5 or 6, which comprises a plurality of amino acid/nucleic acid sequences corresponding to SEQ ID No. 1 homologues from different species or strains of *Eimeria* species parasites which are capable of inducing a protective immune response in a subject against subsequent challenge with an apicomplexan parasite.

8. A vaccine according to any of claims 5 to 7, for the treatment and/or prevention of a disease associated with a parasite of the *Eimeria* species, which also comprises:
(i) polypeptide from an apicomplexan parasite comprising the amino acid sequence as shown as SEQ ID No. 3 or a homologue thereof from different species or strains of *Eimeria* species parasites which are capable of inducing a protective immune response in a subject against subsequent challenge with an apicomplexan parasite; and/or
(ii) a nucleic acid sequence capable of encoding such a polypeptide.

9. A vaccine according to claim 8, wherein the disease is coccidiosis.

10. A vaccine according to claim 8 or 9, which comprises a plurality of amino acid/nucleic acid sequences corresponding to SEQ ID No. 3 homologues from different *Eimeria* strains which are capable of inducing a protective immune response in a subject against subsequent challenge with an apicomplexan parasite.

11. A polypeptide, nucleic acid, vector or vaccine according to any preceding claim, for use in the treatment and/or prevention of a disease.

12. A polypeptide, nucleic acid, vector or vaccine for use according to claim 11 wherein the disease is associated with an *Eimeria* species parasite.

13. A polypeptide, nucleic acid, vector or vaccine for use according to claim 12 wherein the disease is coccidiosis.

## Patentansprüche

1. Polypeptid aus einem Parasiten der Spezies *Eimeria,* umfassend die Aminosäuresequenz gemäß SEQ ID Nr. 1 oder die Eimeria tenella-Aminosäuresequenz, die in Figur 1 (b) als Et IMP-1 dargestellt ist, oder eine Variante mit wenigstens 80% Sequenzidentität mit SEQ ID Nr. 1 und mit der Fähigkeit, eine Immunschutzantwort bei einem Individuum gegen ein nachfolgendes Challenge mit einem Apicomplexa-Parasiten zu induzieren.

2. Nukleinsäure mit der Fähigkeit, ein Polypeptid nach einem vorhergehenden Anspruch zu codieren.

3. Nukleinsäuresequenz nach Anspruch 2, umfassend den codierenden Teil der Nukleotidsequenz gemäß SEQ ID Nr. 2 oder 6.

4. Vektor, umfassend eine Nukleinsäure nach Anspruch 2 oder 3.

5. Impfstoff zur Behandlung und/oder Vorbeugung einer Krankheit in Verbindung mit einem Parasiten der Spezies *Eimeria,* der Folgendes umfasst:
(i) ein Polypeptid nach Anspruch 1; und/oder
(ii) eine Nukleinsäuresequenz mit der Fähigkeit, ein solches Polypeptid zu codieren.

6. Impfstoff nach Anspruch 5, wobei es sich bei der Krankheit um Kokzidiose handelt.

7. Impfstoff nach Anspruch 5 oder 6, der mehrere Aminosäure-/Nukleinsäuresequenzen umfasst, die Homologen von SEQ ID Nr. 1 aus unterschiedlichen Spezies oder Stämmen von Parasiten der Spezies *Eimeria* entsprechen, die zum Induzieren einer Immunschutzantwort bei einem Individuum gegen ein nachfolgendes Challenge mit einem Apicomplexa-Parasiten fähig sind.

8. Impfstoff nach einem der Ansprüche 5 bis 7 zur Behandlung und/oder Vorbeugung einer Krankheit in Verbindung mit einem Parasiten der Spezies *Eimeria,* der auch Folgendes umfasst:
(i) Polypeptid aus einem Apicomplexa-Parasiten, das die Aminosäuresequenz gemäß SEQ ID Nr. 3 oder ein Homolog davon aus unterschiedlichen Spezies oder Stämmen von Parasiten der Spezies *Eimeria,* die zum Induzieren einer Immunschutzantwort bei einem Individuum gegen ein nachfolgendes Challenge mit einem Apicomplexa-Parasiten fähig sind, umfasst; und/oder
(ii) eine Nukleinsäuresequenz mit der Fähigkeit, ein solches Polypeptid zu codieren.

9. Impfstoff nach Anspruch 8, wobei es sich bei der Krankheit um Kokzidiose handelt.

10. Impfstoff nach Anspruch 8 oder 9, der mehrere Aminosäure-/Nukleinsäuresequenzen umfasst, die Homologen von SEQ ID Nr. 3 aus unterschiedlichen *Eimeria*-Stämmen entsprechen, die zum Induzieren einer Immunschutzantwort bei einem Individuum gegen ein nachfolgendes Challenge mit einem Apicomplexa-Parasiten fähig sind.

11. Polypeptid, Nukleinsäure, Vektor oder Impfstoff nach einem vorhergehenden Anspruch zur Verwendung bei der Behandlung und/oder Vorbeugung einer Krankheit.

12. Polypeptid, Nukleinsäure, Vektor oder Impfstoff zur Verwendung nach Anspruch 11, wobei die Krankheit in Verbindung mit einem Parasiten der Spezies *Eimeria* steht.

13. Polypeptid, Nukleinsäure, Vektor oder Impfstoff zur Verwendung nach Anspruch 12, wobei es sich bei der Krankheit um Kokzidiose handelt.

## Revendications

1. Polypeptide issu d'un parasite de l'espèce *Eimeria* comprenant la séquence d'acides aminés présentée sous la dénomination SEQ ID No. 1 ou la séquence d'acides aminés d'*Eimeria tenella* présentée sous la dénomination Et IMP-1 sur la Figure 1 (b) ou une variante présentant une identité de séquences d'au moins 80 % vis-à-vis de SEQ ID NO: 1 et étant capable d'induire une réponse immunitaire protectrice chez un sujet contre une présentation ultérieure d'un parasite apicomplexé.

2. Acide nucléique pouvant coder un polypeptide selon l'une quelconque des revendications précédentes.

3. Séquence d'acides nucléiques selon la revendication 2, comprenant la partie codante de la séquence nucléotidique présentée sous la dénomination SEQ ID No. 2 ou 6.

4. Vecteur comprenant un acide nucléique selon la revendication 2 ou 3.

5. Vaccin destiné au traitement prophylactique et/ou thérapeutique d'une maladie associée à un parasite de l'espèce *Eimeria,* qui comprend :
(i) un polypeptide selon la revendication 1 ; et/ou
(ii) une séquence d'acides nucléiques pouvant coder un tel polypeptide.

6. Vaccin conforme à la revendication 5, où la maladie est la coccidiose.

7. Vaccin selon la revendication 5 ou 6, qui comprend une multitude de séquences d'acides aminés/d'acides nucléiques correspondants à des homologues de SEQ ID No. 1 issus de différentes espèces ou souches de parasite de l'espèce *Eimeria* capables d'induire une réponse immunitaire protectrice chez un sujet contre une présentation ultérieure d'un parasite apicomplexé.

8. Vaccin selon l'une quelconque des revendications 5 à 7, destiné au traitement prophylactique et/ou thérapeutique d'une maladie associée à un parasite de l'espèce *Eimeria,* qui comprend également :
(i) un polypeptide issu d'un parasite apicomplexé comprenant la séquence d'acides aminés présentée sous la dénomination SEQ ID No. 3 ou un homologue de celle-ci issu d'espèces ou de souches différentes de parasite de l'espèce *Eimeria* qui sont capables d'induire une réponse immunitaire protectrice chez un sujet contre une présentation ultérieure d'un parasite apicomplexé; et/ou
(ii) une séquence d'acides nucléiques pouvant coder un tel polypeptide.

9. Vaccin conforme à la revendication 8, où la maladie est la coccidiose.

10. Vaccin selon la revendication 8 ou 9, qui comprend une multitude de séquences d'acides aminés/d'acides nucléiques correspondants à des homologues de SEQ ID No. 3 issus de différentes souches d'*Eimeria* capables d'induire une réponse immunitaire protectrice chez un sujet contre une présentation ultérieure d'un parasite apicomplexé.

11. Polypeptide, acide nucléique, vecteur ou vaccin selon l'une quelconque des revendications précédentes, pour utilisation dans le traitement prophylactique et/ou thérapeutique d'une maladie.

12. Polypeptide, acide nucléique, vecteur ou vaccin pour utilisation selon la revendication 11, où la maladie est associée à un parasite de l'espèce *Eimeria.*

13. Polypeptide, acide nucléique, vecteur ou vaccin pour utilisation selon la revendication 12, où la maladie est la coccidiose.
